# EUROPEAN PATENT SPECIFICATION

(11) **EP 1 071 658 B1**
(45) Date of publication and mention of the grant of the patent: **16.06.2004**
(21) Application number: 99918673.7
(22) Date of filing: 19.04.1999
(51) Int. Cl.: C07C 255/23, A61K 31/275, C07D 311/16

(54) **BTK INHIBITORS AND METHODS FOR THEIR IDENTIFICATION AND USE**
BTK INHIBITOREN UND VERFAHREN ZUR IDENTIFIZIERUNG UND VERWENDUNG
INHIBITEURS BTK ET LEURS PROCEDES D'IDENTIFICATION ET D'UTILISATION

(30) Priority: 17.04.1998 US 82094 P; 19.03.1999 US 273191
(43) Date of publication of application: 31.01.2001
(73) Proprietor: Parker Hughes Institute, St. Paul, MN 55113 (US)
(72) Inventor: UCKUN, Fatih, M., White Bear Lake, MN 55110 (US); ZHENG, Yaguo, Roseville, Minnesota 55113 (US); GHOSH, Sutapa, Shoreview, MN 55126 (US)
(74) Representative: Plougmann & Vingtoft A/S
(86) International application number: PCT/US1999/008556
(87) International publication number: WO 1999/054286

(56) References cited:
- EP-A- 0 537 742
- EP-A- 0 551 230
- EP-A- 0 652 214
- WO-A-91/17748
- WO-A-94/14789
- WO-A-96/26934
- WO-A-96/31206
- WO-A-98/25608
- DE-A- 2 555 685
- US-A- 5 489 697
- US-A- 5 700 823
- GHOSH S ET AL: "ALPHA-CYANO-BETA-HYDROXY-BETA-METHYL-N-U4 -(TRIFLUOROMETHOXY)PHENYL PROPENAMIDE: AN INHIBITOR OF THE EPIDERMAL GROWTH FACTOR RECEPTOR TYROSINE KINASE WITH POTENT CYTOTOXIC ACTIVITY AGAINST BREAST CANCER CELLS" CLINICAL CANCER RESEARCH,US,THE ASSOCIATION, DENVILLE, NJ, vol. 4, no. 11, November 1998 (1998-11), pages 2657-2668, XP000856462 ISSN: 1078-0432
- MAHAJAN S ET AL: "RATIONAL DESIGN AND SYNTHESIS OF A NOVEL ANTI-LEUKEMIC AGENT TARGETING BRUTON'S TYROSINE KINASE (BTK), LFM-A13 ALPHA-CYANO-BETA-HYDROXY-BETA-METHYL-N-(2, 5-DIBROMOPHENYL)PROPENAMIDE" JOURNAL OF BIOLOGICAL CHEMISTRY,US,AMERICAN SOCIETY OF BIOLOGICAL CHEMISTS, BALTIMORE, MD, vol. 274, no. 14, 2 April 1999 (1999-04-02), pages 9587-9599, XP000856755 ISSN: 0021-9258
- AHLUWALIA, V. K. ET AL: "Formation of 4-ethylcoumarins from.beta.-diketones - reaction mechanism" INDIAN J. CHEM., SECT. B (1977), 15B(3), 240-1 ,1977, XP000889920
- CROMBIE, LESLIE ET AL: "Synthesis of mammeins and surangin A" TETRAHEDRON LETT. (1985), 26(24), 2929-32 ,1985, XP002132461
- CHEMICAL ABSTRACTS, vol. 88, no. 21, 22 May 1978 (1978-05-22) Columbus, Ohio, US; abstract no. 152354, REHSE, KLAUS ET AL: "Coumarin derivatives related to calophyllolid" XP002132462 & ARCH. PHARM. (WEINHEIM, GER.) (1978), 311(1), 52-8 ,1978,

## Description

### Field of the Invention

This invention relates to the use of inhibitors of the family tyrosine kinase, and particularly, inhibitors of Bruton's Tyrosine Kinase (BTK).

### Background of the Invention

Apoptosis is a common mode of eukaryotic cell death that is triggered by an inducible cascade of biochemical events leading to activation of endonucleases that cleave the nuclear DNA into oligonucleosome-length fragments. Several of the biochemical events that contribute to apoptotic cell death as well as both positive and negative regulators of apoptosis have recently been identified (Whyllie, A., et al. (1980) *Int Rev Cytol* 68, 251-305; Steller, H., (1995) *Science* 267, 1445-1449; Fraser, A., Evan, G. (1996) *Cell* 85, 781-784; and Korsmeyer, S.J. (1995) *Trends Genet* 11, 101 - 105). Apoptosis plays a pivotal role in the development and maintenance of a functional immune system by ensuring the timely self-destruction of autoreactive immature and mature lymphocytes as well as any emerging target neoplastic cells by cytotoxic T cells.

In addition to the beneficial effects associated with apoptosis, inappropriate apoptosis contributes to the pathogenesis and drug resistance of human leukemias and lymphomas (Cohen, J.J., et al. (1992) *Annu Rev Immunol* 10, 267-293; Linette, G.P. and Korsmeyer, S.J. (1994) *Curr Opin Cell Biol.* 6, 809-815; and Thompson, C.B. (1995) *Science* 367, 1456-1462). Thus, agents that are useful to modulate apoptosis are potentially useful as therapeutic agents for treating diseases in which inappropriate apoptosis is implicated. As a result, there is a considerable amount of ongoing research devoted to the identification of molecular regulators of apoptosis, and there is currently a need for novel agents (e.g. chemical or biological), and novel therapeutic methods, that are useful for modulating apoptosis. Such agents and methods may be useful for treating cancer (e.g. leukemias and lymphomas) or immune disorders in mammals. They may also be useful as pharmacolocical tools for use in *in vitro* or *in vivo* studies to enhance the understanding of the molecular basis of apoptosis (e.g. the pro-apoptotic versus the anti-apoptotic regulatory signal), as well as the pathogenesis of human lymphoid malignancies.

WO-A-9117748 discloses use of isoxazole-4-carboxamide derivatives and hydroxyalkylidene-cyanoacetamide for the treatment of cancer diseases and rheumatic diseases. DE-A-2555685 discloses cyanoacidanilide derivatives and the preparation thereof. EP-A-537742 discloses styrene derivatives having an inhibitory activity on tyrosine-specific protein kinase and inhibitory activity on cancer cell proliferation. Ghosh et al., Clinical Cancer Research, November 1998, vol. 4, 2657-68, disclose α-Cyano-β-hydroxy-β-methyl-N-[4-(trifluoromethoxy)phenyl] propenamide for inhibition of the Epidermal Growth Factor Receptor Tyrosine Kinase, with potent cytotoxic activity, against breast cancer cells.

BTK is a dual-function regulator of apoptosis that promotes radiation-induced apoptosis but inhibits Fas-activated apoptosis in B-cells. (Uckun, F.M. Commentary: Burton's Tyrosine Kinase (BTK) as a Dual-Function Regulator of Apoptosis; *see also* Uckun, F.M. et al., BTK as a Mediator of Radiation-induced Apoptosis in DT-40 Lymphoma B-Cells, *Science* 273: 1096-1100 (1996). BTK functions in a pro-apoptotic manner when B-cells are exposed to reactive oxygen intermediates, at least in part, by downregulating the anti-apoptotic activity of STAT3 transcription factor. In contrast, BTK associates with the death receptor Fas, impairs its interaction with FADD, which is essential for the recruitment and activation of FLICE by Fas during the apoptotic signal, thereby preventing the assembly of a proapoptotic death inducing signaling complex (DISC) after Fas-ligation.

### Summary of the Invention

The invention provides the use of compounds of formula I: 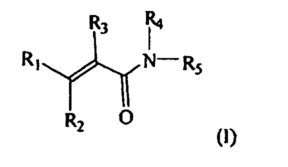 where:
R₁ is (C₁-C₃)alkyl, (C₃-C₆)cycloalkyl, phenyl, or NRₐR_{b};
R₂ is hydroxy, (C₁-C₆)alkoxy, (C₁-C₆)alkanoyloxy, amino (C₂-C₅)alkoxy; hydroxy (C₂-C₅)alkoxy, amino (C₂-C₅)alkanoyloxy; or hydroxy (C₂-C₅) alkanoyloxy;
R₃ is cyano or (C₁-C₃)alkanoyl;
R₄ is hydrogen, (C₁-C₃)alkyl; hydroxy (C₂-C₅)alkyl; or amino (C₂-C₅)alkyl;
R₅ is phenyl substituted by -S(O)₂R_{c} or by halo and at least one other substituent;
Rₐ and R_{b} are each independently hydrogen, or (C₁-C₃)alkyl; or Rₐ and R_{b} together with the nitrogen to which they are attached are pyrrolidino, piperidino, morpholino, or thiomorpholino;
wherein any aryl, or heteroaryl of R₁ and R₅ is optionally substituted with one or more (e.g. 1, 2, or 3) substituents independently selected from halo, nitro, cyano, hydroxy, trifluoromethyl, trifluoromethoxy, (C₁-C₃)alkoxy, (C₁-C₃)alkyl, (C₁-C₃)alkanoyl, -S(O)₂R_{c}, or NRₐR_{b;} wherein R_{c} is (C₁-C₃)alkyl, or aryl or a pharmaceutically acceptable salt thereof as described in claim 1.

The compounds of the invention are designed to fit a composite binding pocket model of the BTK domain, having a molecular volume of less than the volume of the binding pocket (e.g., less than about 600Å³) and preferably a volume that approaches 2/3 the volume of the pocket, e.g., approximately 400Å³. Most preferably, the inhibitors of the invention are designed to fill the space of the binding pocket and to interact with residues of the pocket for enhanced binding.

The invention provides the use of a compound of formula I for the manufacture of a medicament for the treatment of a pathological condition or a disease in a mammal, such as human, which is associated with BTK activity. These conditions or disorders are selected from B-cell lymphoproliferative disorders/autoimmune diseases, mast cell disorders, conditions that relate to improper platelet aggregation, and rejection of xenotransplants.

### Brief Description of the Figures

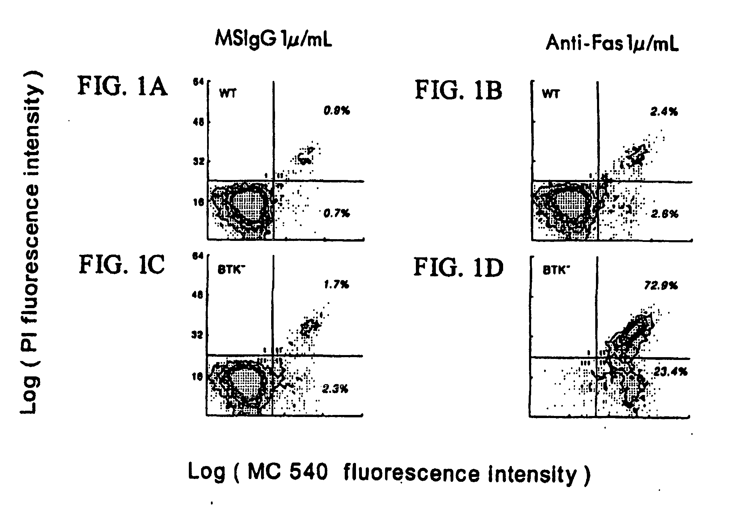

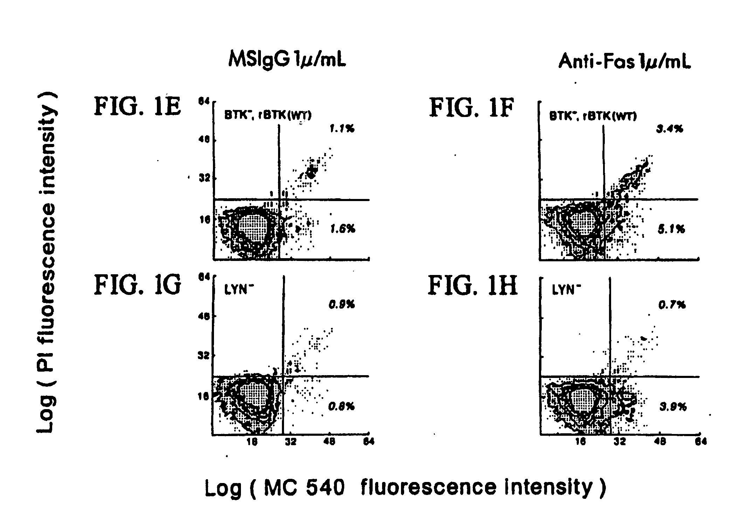

Figure 1: BTK is an inhibitor of Fas-mediated apoptosis in DT-40 lymphoma B cells. FACS correlated two-parameter displays of wild-type (WT), BTK-deficient (BTK-), LYN-deficient (LYN-) DT-40 cells as well as BTK-deficient DT-40 cells reconstituted with wild-type human *btk* gene (BTK; rBTK[WT]) stained with MC540 and PI 24 hours after treatment with the control mouse IgG MsIgG (1 µg/mL) or anti-Fas (1 µg/mL). The percentages indicate the fraction of cells at an early stage of apoptosis, as measured by single MC540 fluorescence, and the fraction of cells at an advanced stage of apoptosis, as measured by dual MC540/PI fluorescence (Uckun, F.M., et al. (1996) *Science* 273, 1096-1100).

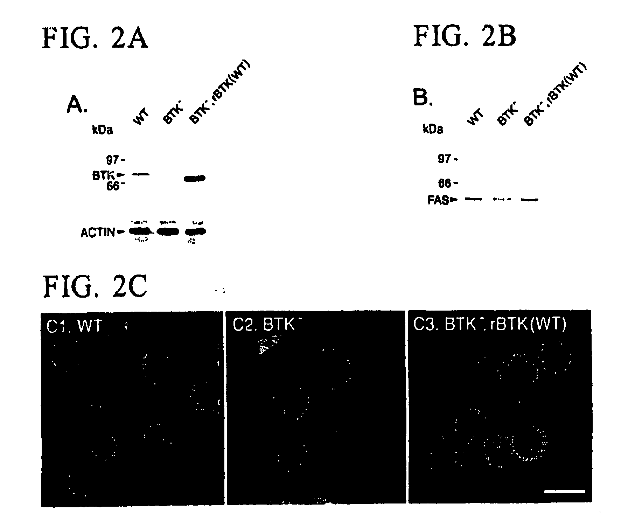

Figures 2A-2C: Fas protein expression levels in wild-type and BTK-deficient DT-40 cells. Figure 2A shows the expression levels of BTK and ACTIN in wild-type, BTK-deficient, and human btk gene-reconstituted BTK-deficient DT-40 cells were measured by Western blot analysis using appropriate monoclonal antibodies and the ECL chemiluminescence detection system (Amersham Life Sciences, used according to the manufacturer's recommendations) (Dibirdik, I., et al. (1998) *J. Biol. Chem,* 273, 4035-4039; Uckun, F.M., et al. (1997) *Blood,* 89, 3769-3777). Figure 2B shows membranes immunoblotted with anti-BTK and anti-ACTIN antibodies that were stripped and reblotted with the monoclonal anti-Fas antibody to compare the Fas protein expression levels in the individual clones. Figure 2C hows Fas expression levels of WT and BTK-deficient DT-40 cells examined by confocal microscopy. Green: anti-Fas labeling; Blue: Toto-3 stained DNA in nucleus; Scale Bar = 10 mm.

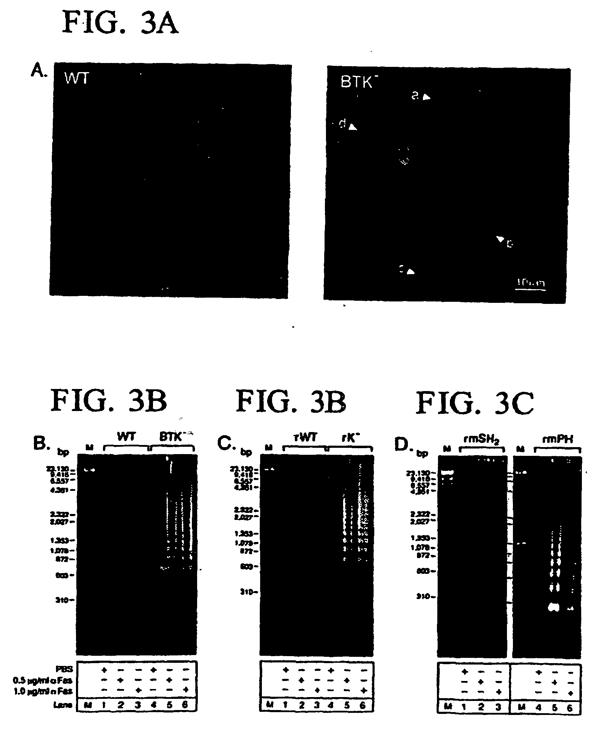

Figures 3A-3D: BTK inhibits Fas-mediated apoptosis. Figure 3A is a photograph showing wildtype cells (WT) and BTK-deficient (BTK-) DT-40 cells were treated for 24 hours with 1 µg/ml anti-Fas, co-stained with a rabbit polyclonal anti-tubulin antibody (green fluorescence) and the DNA specific dye toto-3 (blue fluorescence), and examined by laser scanning confocal microscopy, as described in the Examples. Unlike WT cells, the majority of BTK- cells show apoptotic changes including nuclear fragmentation (a,b,d) and shrinkage (c). Bar = 10mm. Figure 3B is a DNA gel showing WT and BTK- DT-40 cells exposed to anti-Fas antibody as detailed in the Examples, harvested and DNA from Triton-X-100 lysates was analyzed for fragmentation, as described (Uckun, F.M., et al. (1996) *Science* 273, 1096-1100). Figures 3C and 3D show BTK-deficient DT-40 cells reconstituted with wild-type (rWT), kinase domain mutant (rK-), SH2-domain mutant (rmSH2), or PH-domain mutant (rmPH) forms of the human *btk* gene were examined for sensitivity to anti-Fas antibody-induced apoptosis as described in the Examples. Controls were treated with PBS in culture medium for 24 hours at 37°C and 5% CO₂ prior to harvesting.

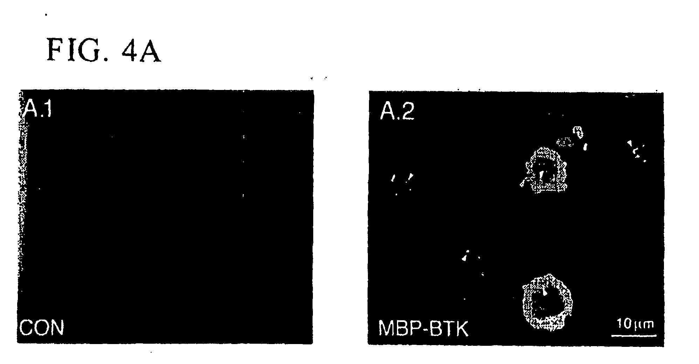

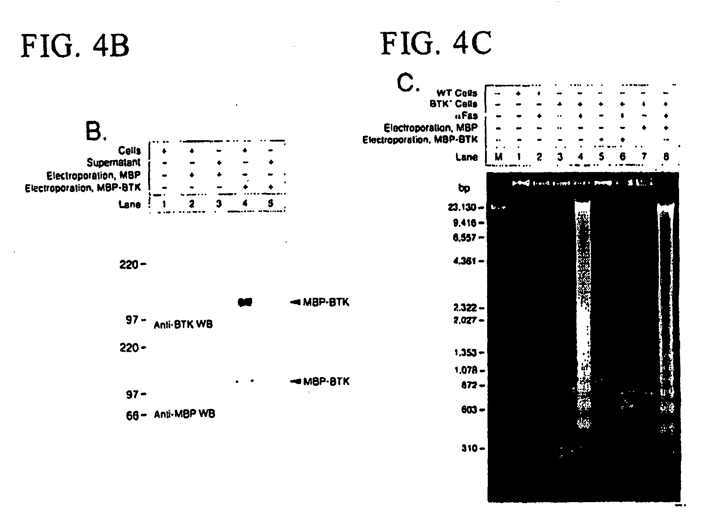

Figures 4A-4C: Anti-apoptotic properties of BTK confirmed by BTK protein reconstitution of BTK- DT40 cells. Maltose binding protein (MBP) or MBP-BTK were electroporated into BTK- DT-40 cells prior to treatment with anti-Fas antibody, as described in the Examples. Figure 4A is a photograph showing MBP-BTK-electroporated BTK-deficient DT-40 cells and non-electroporated BTK-deficient DT-40 cells labeled with an antibody raised against MBP. The secondary antibody was a FITC-conjugated goat anti-rabbit antibody. Cells were analyzed using a Bio-Rad MRC-1024 Laser Scanning Confocal Microscope Digital images were processed using Adobe Photoshop software and printed using a Fuji Pictography Printer. There was no significant staining above background in control non-electroporated cells (Figure 4A.1). Arrowheads indicate MBP antibody reactive material in the cytoplasm of cells electroporated with the MBP-BTK fusion protein (Figure 4A.2). Two populations were observed in the MBP-BTK electroporated cells. Some cells had very bright labelling at the periphery of the cell, while other cells had large punctate staining inside the cytoplasm. Green=MBP, Bar=10 mm. Figure 4B shows a Western blot. Lysates as well as supernatants of BTK-deficient DT-40 cells electroporated with either MBP or MBP-BTK were subjected to Western blot analysis using anti-BTK and anti-MBP antibodies as described in Experimental Procedures. The 115 kDa MBP-BTK fusion protein reactive with both antibodies was detected only in lysates (but not supernatants) from MBP-BTK electroporated cells. Figure 4C is a gel cells were harvested 24 hours after exposure to anti-Fas antibody and DNA from Triton-X-100 lysates was analyzed for fragmentation, as described by Uckun, F.M., et al. (1996) *Science* 273, 1096-1100; and Uckun, F.M., et al. (1995) *Science* 267, 886-91). Anti-Fas treatment induced apoptosis in BTK deficient cells but not in WT cells or BTK deficient cells into which MBP-BTK was electroporated. Electroporation of MBP (negative control) had no effect on apoptosis.

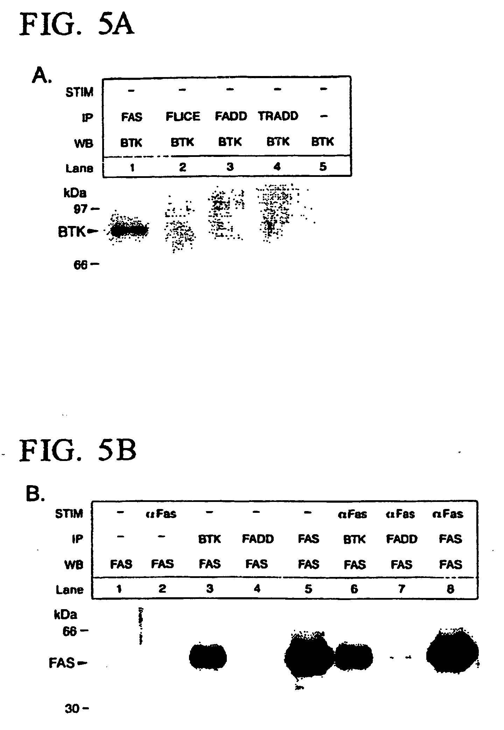

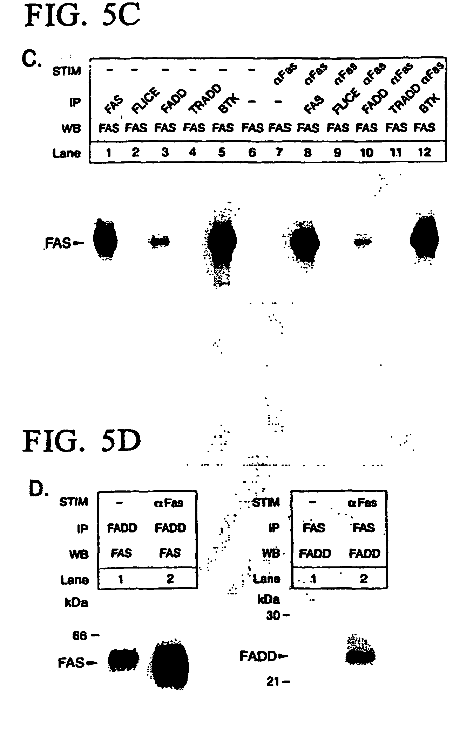

Figures 5A-5D: BTK associates with FAS and Interferes with FAS-FADD Interactions. The Fas, FLICE, FADD, and TRADD immune complexes immunoprecipitated from Nonidet P-40 lysates of untreated wild-type DT-40 lymphoma B-cells were collected, washed, boiled in 2x SDS sample buffer, fractionated on 12.5% polyacrylamide gels, transferred to an Immobilon-PVDF membrane, and examined for the presence of BTK protein by immunoblotting, as described in the Examples and shown in Figure 5A. The BTK and FADD immune complexes (as well as the positive control FAS immune complexes) immunoprecipitated from untreated versus Fas-activated BTK-deficient DT-40 lymphoma B-cells, which were reconstituted with wild-type human *btk* gene, were collected, washed, boiled in 2x SDS sample buffer, fractionated on 12.5% polyacrylamide gels, transferred to an Immobilon-PVDF membrane, and immunoblotted with a monoclonal anti-Fas antibody, as described below and shown in Figure 5B. FAS, FLICE, FADD, TRADD, and BTK immune complexes from lysates of BTK-positive NALM-6-UM human B-cell precursor leukemia cells were subjected to anti-Fas Western blot analysis as shown in Figure 5C. FADD was immunoprecipitated from Nonidet-P-40 lysates of untreated versus Fas-activated (anti-Fas 1 µg/ml x 1 hour) BTK-deficient DT-40 cells, as described in the Examples. The immune complexes were collected, washed, boiled in 2x SDS sample buffer, fractionated on 12.5% polyacrylamide gels, transferred to an Immobilon-PVDF membrane, and immunoblotted with a monoclonal anti-Fas antibody (Figure 5D). Similarly, the FAS immune complexes from the same cells were examined for the presence of FADD by immunoblotting.

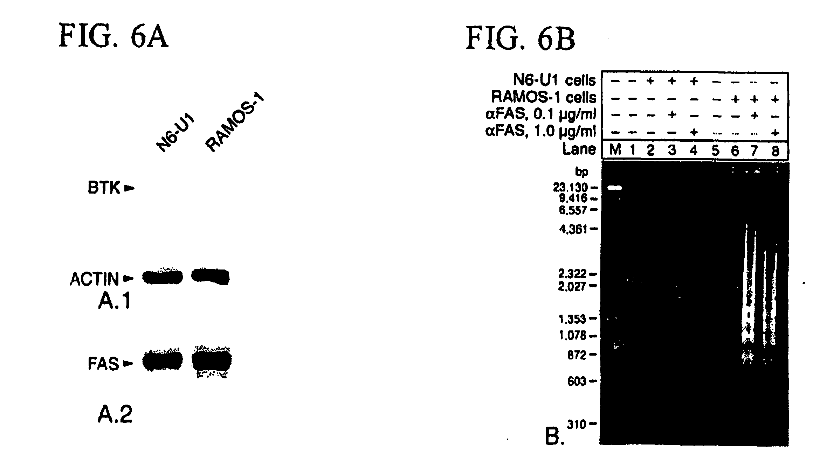

Figures 6A-6D: Apoptotic Responses of Human B-lineage Leukemia Cells to Fas Ligation in Relationship to BTK Expression and Function. Anti-BTK and anti-Actin dual-antibody Western blot analysis of whole cell lysates from BTK-positive NALM-6-UM1 (N6-U1) and BTK-deficient RAMOS-1 cells (Figure 5A). Anti-Fas Western blot analysis of the same cell lysates. [B] Cells were harvested 24 hours after exposure to anti-Fas antibody at 0.1 µg/ml or 1.0 µg/ml concentrations and DNA from Triton-X-100 lysates was analyzed for fragmentation, as described by Uckun, F.M., et al. (1996) *Science* 273, 1096-1100; and Uckun, F.M., et al. (1995) *Science* 267, 886-91. Anti-Fas treatment induced apoptosis in BTK-deficient RAMOS-1 cells but not in BTK-positive N6-U 1 cells. [C.1] and [C.2] Anti-BTK and anti-Fas Western blot analysis of whole cell lysates from N6-U1 cells treated with the BTK inhibitor LMA-13 (10 µM x 4 hours). [C.3] Anti-Fas and Anti-BTK Western blot analysis of anti-BTK antibody immunoprecipitated BTK immune complexes from untreated (= - Inhibitor) and LMA-13-treated (10 µM x 4 hours) (= + Inhibitor) N6-U1 cells. [D]. N6-U1 cells were harvested 24 hours after exposure to anti-Fas antibody at 0.1 µg/ml or 1.0 µg/ml concentrations and DNA from Triton-X-100 lysates was analyzed for fragmentation, as described by Uckun, F.M., et al. (1996) *Science* 273, 1096-1100; and Uckun, F.M., et al. (1995) *Science* 267, 886-91. Anti-Fas treatment induced apoptosis in LMA-13-pretreated (10 µM x 4 hours) (= Inhibitor +) N6-U1 cells. In contrast, no apoptosis was observed in N6-U1 cells that were not pretreated with this BTK inhibitor (= Inhibitor -). An ECL detection system (Amersham Pharmacia Biotech, Arlington Heights, Illinois, cat no. RPN2106) was used for the Western blot analyses in [A.1], [A.2], [C.1], [C.2], and [C.3].

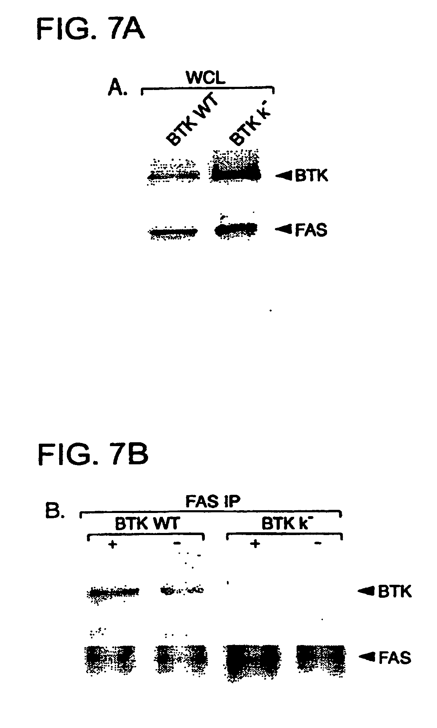

Figures 7A-7B: Kinase-Inactive BTK Does Not Associate with Fas. Anti-BTK (A.1) and anti-Fas (A.2) Western blot analysis of whole cell lysates from BTK-deficient DT-40 cells (Figure 7A) reconstituted with wild-type human BTK (BTK-, rBTK[WT]) (Lane 1) or kinase domain mutant inactive human BTK (BTK-, rBTK[K-] (Lane 2). The expression levels of BTK and Fas were measured by immunoblotting using appropriate antibodies and the ECL chemiluminescence detection system. Figure 7B shows anti-BTK (B.1) and anti-Fas (B.2) Western blot analysis of Fas immune complexes from BTK-, rBTK[WT] cells with (Lane 1) or without (Lane 2) anti-Fas antibody pretreatment and from BTK-, rBTK[K-] cells with (Lane 3) or without (Lane 4) anti-Fas antibody pretreatment. The immune complexes immunoprecipitated from Nonidet P-40 whole cell lysates were collected, washed, boiled in 2x SDS sample buffer, fractionated on 12.5% polyacrylamide gels, transferred to an Immobilon-PVDF membrane, and examined for the presence of BTK and Fas proteins by immunoblotting, as described in the Methods.

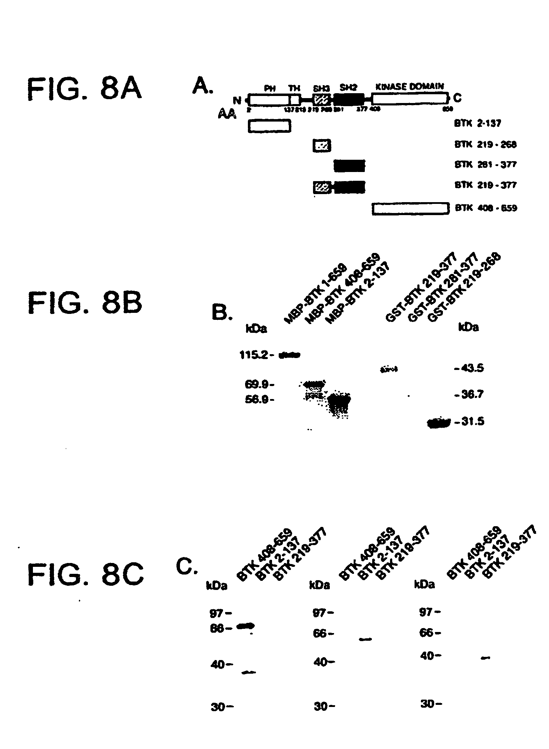

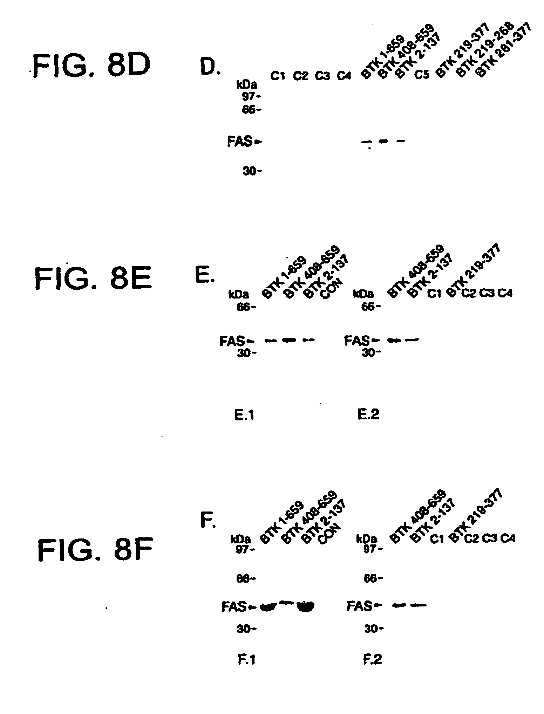

Figures 8A-8F: Binding of BTK fusion proteins to Fas protein in chicken and human B-lineage lymphoid cells. Figure 8A shows schematic diagrams of full-length and truncated MBP- and GST-fusion proteins corresponding to various domains of BTK. The inclusive amino acid (AA) sequence is indicated for each truncation mutant. BTK 1-659, BTK 408-659, BTK 2-137, as well as BTK 519-567 containing Y551 transphosphorylation site within the catalytic domain (used as a control) were fused to MBP. BTK 219-377, BTK 281-377 and BTK 219-268 were fused to GST. Figure 8B shows MBP-BTK and GST-BTK fusion proteins (7.5 µg/lane) analyzed by SDS-PAGE using 12% polyacrylamide gels and visualized by staining of the gels with Coomassie R-250 Blue. Figure 8C is a Western blot analysis of purified BTK from proteins corresponding to the kinase-(BTK 408-659), PH-(BTK 2-137) and SH₂-SH₃-domains (BTK 219-377) of BTK using domain-specific antibodies, as described in the Experimental Procedures. Figures 8D-8F demonstrate functional roles for the kinase and PH domains of BTK in BTK-Fas interactions. [Figure 8D] BTK-deficient DT-40 chicken lymphoma B-cells; [Figure 8E] human NALM-6 pre-B leukemia cells; [Figure 8F]: KL2 human EBV-transformed lymphoblastoid cells. MBP-BTK and GST-BTK fusion proteins were used in pull-down binding assays to examine their ability to interact with Fas in BTK deficient DT-40 cells, as described in the Examples. Fusion protein adsorbates and control samples C1-C5 (C1(=CON): Cell lysate + amylose beads (no fusion protein added); C2: Cell lysate + glutathione-agarose beads (no fusion protein added); C3: MBP-BTK 1-659 + amylose beads (no cell lysate); C4: GST-BTK 219-268 + glutathione-agarose beads (no cell lysate added); C5: MBP-BTK 519-567 + amylose beads + cell lysate) were resolved by SDS-PAGE, immunoblotted with the monoclonal anti-Fas antibody, and developed with ECL.

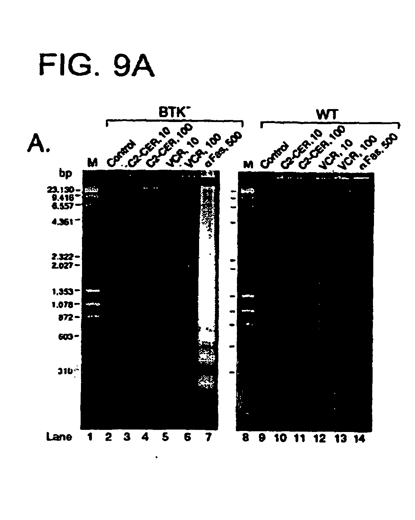

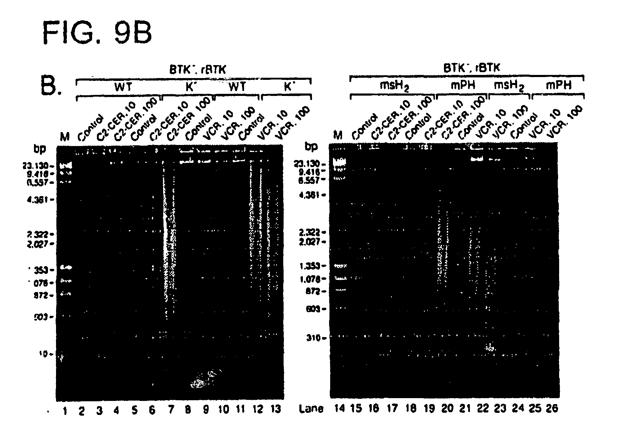

Figures 9A-9B: The anti-apoptotic function of BTK. Wild-type and BTK-deficient (BTK-) DT-40 lymphoma B cells (Figure 9A) as well as BTK- DT-40 cells reconstituted with wild-type or mutant human BTK (Figure 9B) were treated with C2-CER, vincristine (VCR), or anti-Fas antibody, as described in the Examples. BTK-deficient DT-40 (BTK-) cells expressing wild-type BTK, BTK(Arg⁵²⁵ ®Gln), BTK(Arg²⁸®Cys), and BTK(Arg³⁰⁷®Ala) were designated as BTK⁻ ,rBTK(WT), BTK-,rBTK(K-), BTK⁻,rBTK(mPH) and BTK-,rBTK(mSH2), respectively. Vehicle (0.1% DMSO in PBS) treated as well as drug treated cells were maintained in culture medium for 24 hours at 37°C and 5% CO₂ before harvesting. DNA from Triton-X-100 lysates was analyzed for fragmentation as described above. Uckun, F. M., et al. (1996) *Science* 22, 1096-1100.

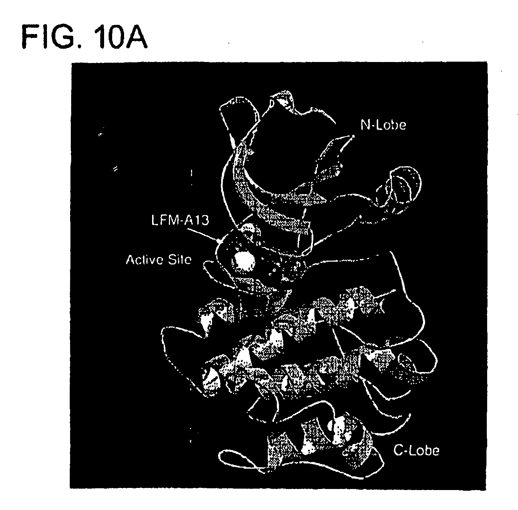

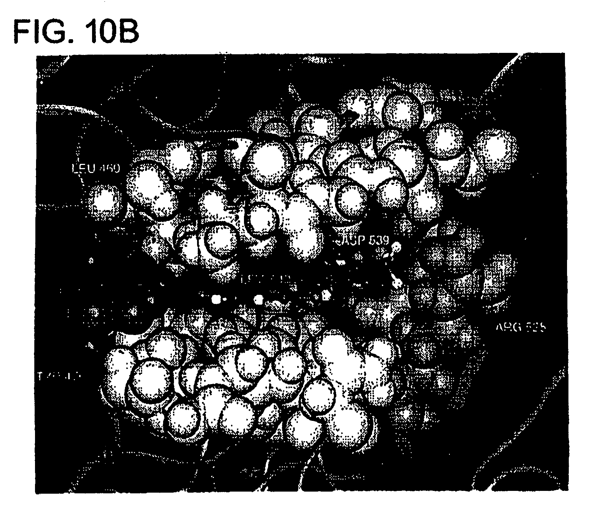

Figures 10A-10B: Homology model of BTK Kinase domain. Figure 10A is a ribbon representation of the homology model of the BTK kinase domain. The LFM-A13 molecule is shown as a space filling model in the catalytic site of BTK. Prepared using Molscript and Raster3D programs (Bacon, D. J., and Anderson, W. F. (1988) *J. Molec. Graphics* 6, 219-20; Kraulis, P. (1991) *J. Appl. Cryst.* 24, 946-50; and Merritt, E. A., and Murphy, M. E. P. (1994) *Acta Cryst.* D50, 869-73). Figure 10B is a space filling representation of the backbone of the catalytic site residues of the BTK kinase domain. The C-alpha chain of BTK is represented as a blue ribbon. Shown in yellow, green, pink, and blue are the residues at the four comers of the rectangular shaped binding pocket. A ball and stick model of the BTK inhibitor LFM-A13 is shown in multicolor and represents the favorable orientation of this molecule in the kinase active site of BTK. Prepared using InsightII program ((1996), Molecular Simulations, Inc., San Diego, CA).

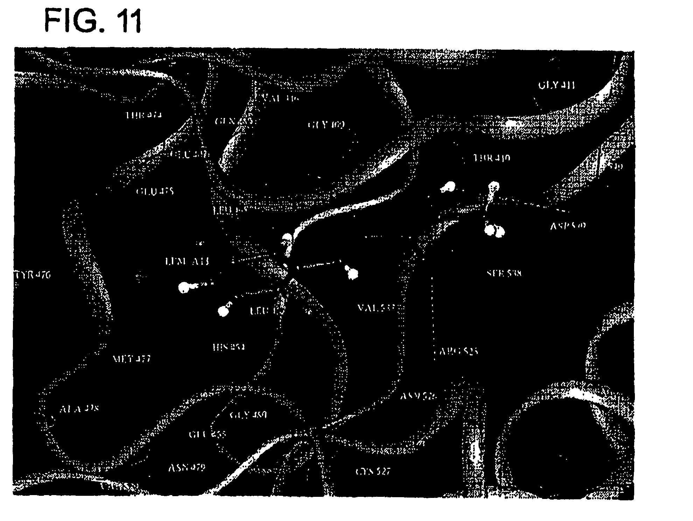

Figure 11: Docked position of the LFM-A13 molecule (multi-color) at the catalytic site (blue ribbon) of the kinase domain of BTK. Dashed lines represent hydrogen bonds between LFM-A13 and the kinase domain residues of BTK.

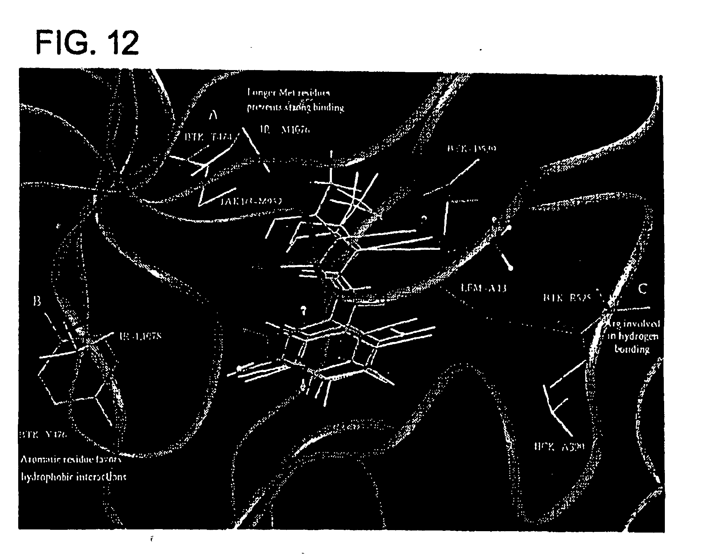

Figure 12: Superimposed docked positions of LFM (purple), LFM-A 12 (red) and LFM-A13 (multi-color) in the catalytic site (blue ribbon) of the kinase domain of BTK. Docked positions of LFM and LFM-A12 are shown for comparative purposes only.

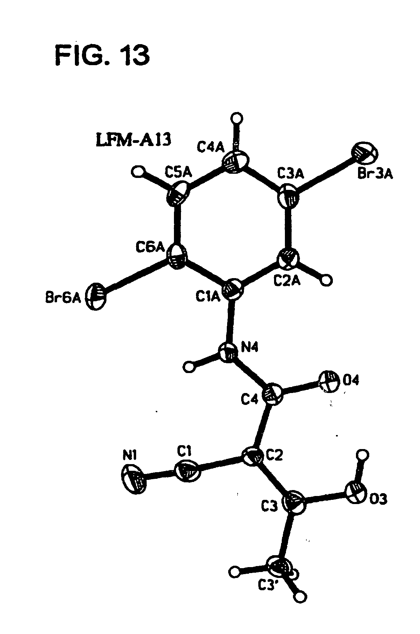

Figure 13: ORTEP picture of the crystal structure of the BTK inhibitor, LFM-A13.

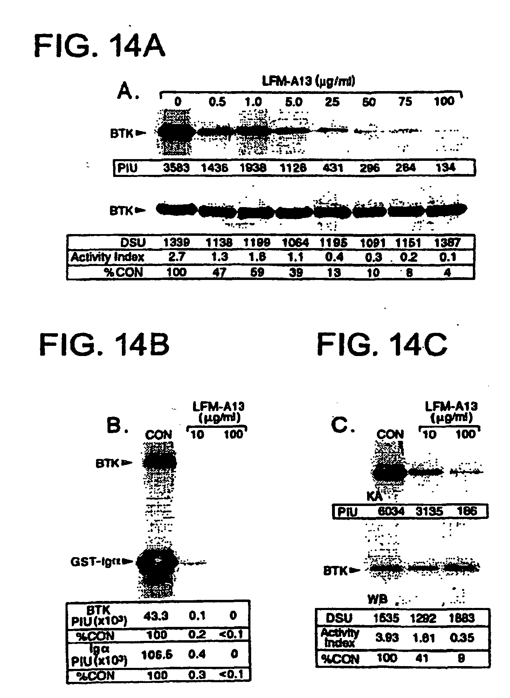

Figures 14A-14C: Effects of LFM-A13 on the Tyrosine Kinase Activity of BTK. A highly purified (>90%) preparation of BTK produced in a baculovirus vector expression system was treated for 1 hour at room temperature with LFM-A 13 at the indicated concentrations. The enzymatic activity of BTK shown in Figure 14A was determined by measuring autophosphorylation in a 10 minute kinase assay, as described in the Examples. BTK was immunoprecipitated from B18.2 cells (i.e., BTK- DT-40 cells reconstituted with wild-type human BTK), treated with LFM-A13 or vehicle (0.1% DMSO in PBS) for 1 hour, and then assayed for PTK activity, measured by autophosphorylation as well as phosphorylation of GST-Iga, which was used an exogenous kinase substrate. The kinase data are shown in Figure 14B. B18.2 lymphoma B-cells were treated with LFM-A 13, then lysed, and BTK immune complex kinase assays and Western blots were performed as described in the Examples. The data are shown in Figure 14C. PIU: Phosphoimager units; DSU, densitometric scanning units; CON, control.

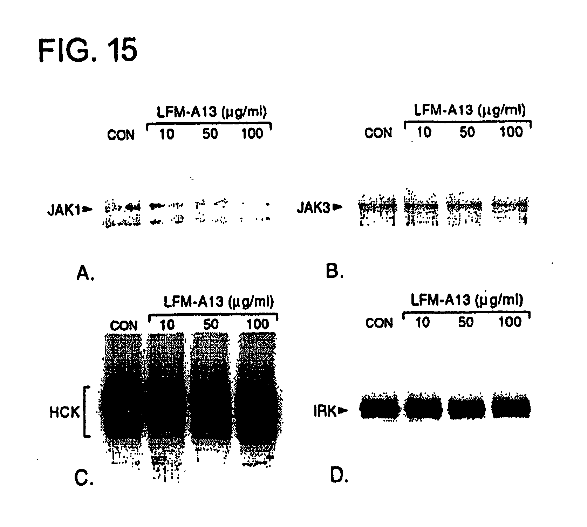

Figure 15: Effects of LFM-A13 on the Tyrosine Kinase Activity of JAK1, JAK3, HCK, and IRK. JAK1 and JAK3 immunoprecipitated from Sf21 insect ovary cells transfected with the appropriate baculovirus expression vectors, HCK immunoprecipitated from COS-7 cells transfected with the pSV7c-HCK plasmid, and IRK immunoprecipitated from HepG2 hepatoma cells were treated with LFM-A13, then subjected to *in vitro* kinase assays as described in Experimental Procedures.

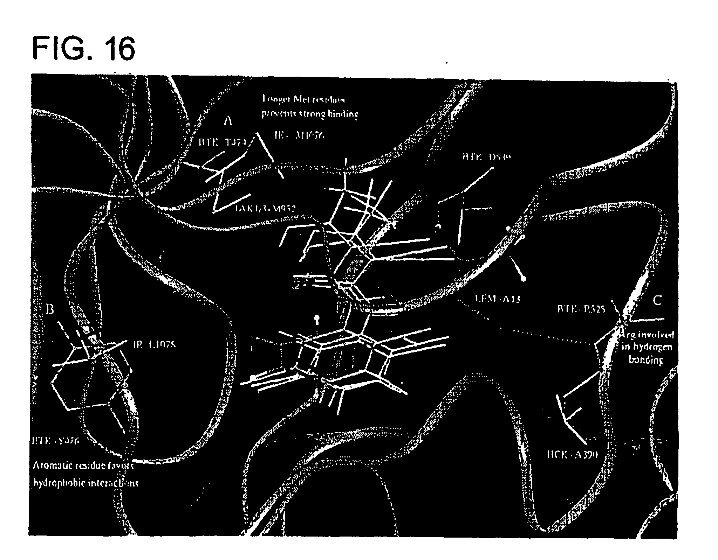

Figure 16: Structural Basis for the Selectivity of LFM-A13 for BTK. Shown in light blue is a trace of the BTK homology model with selected residues at positions A, B, and C, together with the docked position of the leflunomide metabolite analog LFM-A13 (multicolor). Shown in red is the docked position of LFM-A13 with a model of EGFR and the residue difference between EGFR and BTK at position B. Shown in yellow is the docked position of LFM-A13 with the crystal structure of HCK and the residue difference between HCK and BTK at position C. Shown in pink is the docked position of LFM-A13 with models of JAK3/JAK1 and the residue difference between JAK3/JAK1 and BTK at position A. Shown in dark blue is the docked position of LFM-A 13 with the crystal structure of IRK and the residue differences between IRK and BTK at positions A and B.

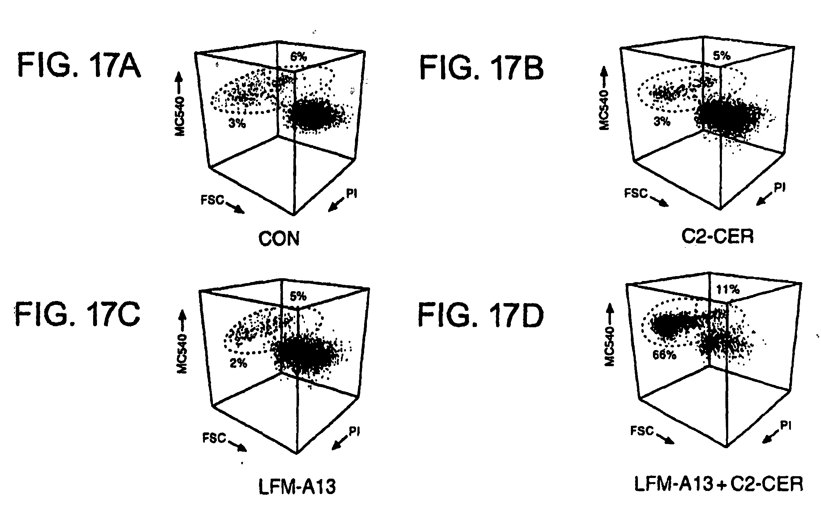

Figure 17: Effects of LFM-A13 on Ceramide-Sensitivity of Human Leukemia Cells. FACS correlated three-parameter (FSC, forward scatter=size; fluorescence from PI, propidium iodide, and fluorescence from MC540 staining) displays of ALL-1 Ph/t(9;22)⁺ human ALL cells stained with MC540 and PI 24 hours after treatment with vehicle (0.1% DMSO in PBS), C2-Ceramide (C2-CER) (10 µM), LFM-A13 (200 µM) or LFM-A13 + C2-CER. The percentages indicate the fraction of cells at an early stage of apoptosis, as measured by single MC540 fluorescence, and the fraction of cells at an advanced stage apoptosis, as measured by dual MC540/PI fluorescence.

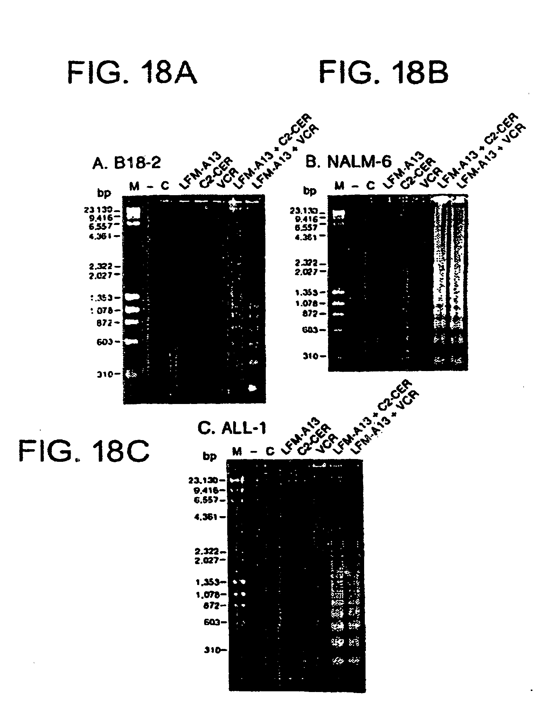

Figures 18A-18C: Chemosensitizing Effects of LFM-A13. BTK-deficient DT-40 cells reconstituted with wild-type human BTK gene (i.e., B18.2 clone) (Figure 18A), NALM-6 human pre-B ALL cells (Figure 18B), and ALL-1 human Ph⁺ ALL cells (Figure 18C) were treated with LFM-A13 (100 µM), vincristine (VCR) (10 ng/ml), C2-Ceramide (C2-CER) (10 µM), LFM-A13 (100 µM) + VCR (10 ng/ml), LFM-A 13 (100 µM) + C2-CER (10 µM) for 24 hours at 37°C. DNA from Triton-X-100 lysates was analyzed for fragmentation, as described by Uckun, F. M., et al. (1996) *Science* 22, 1096-1100.

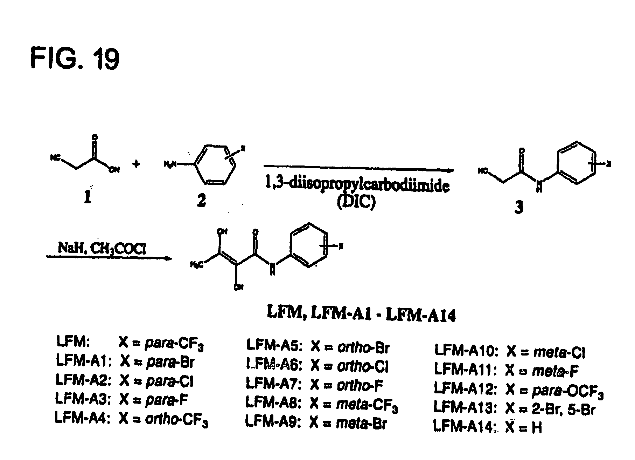

Figure 19: Illustrates the synthesis of LFM-13 and other related compounds. LFM, LFM-A1 to LFM-A12, and LFM-A14 are included for comparative purposes only.

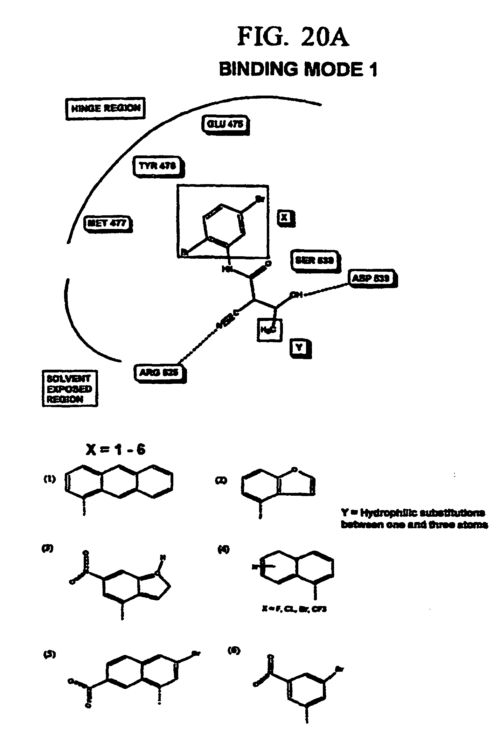

Figures 20A-20C: Illustrate the binding interactions of LFM-13 with the BTK binding pocket.

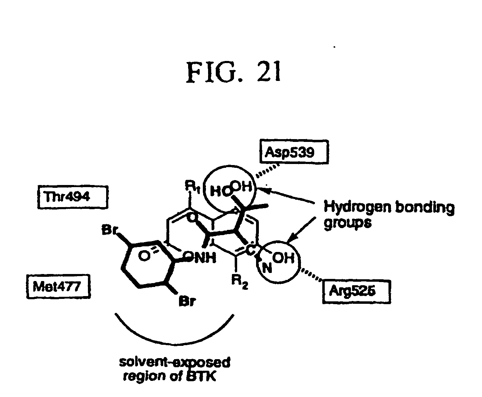

Figure 21: Binding features of both 5,7-Dihydroxy-8-propanoyl-4-propyl-2H-1-benzopyran-2-one and LFM-A13. Based on docking studies these compounds fit into the ATP-binding site of BTK. Both compounds contain hydrogen binding groups that can interact with Arg 525 and Asp 539. Binding features of 5,7-Dihydroxy-8-propanoyl-4-propyl-2H-1-benzopyran-2-one are shown for comparative purposes only.

### Detailed Description of the Invention

The Fas/APO-1 (CD95) cell surface receptor, a member of the tumor necrosis factor (TNF) receptor family, is one of the major regulators of apoptosis in a variety of cell types. Functional abnormalities of Fas have been associated with pathologic conditions of the immune system homeostasis, including lymphoproliferative disorders, immunodeficiencies and autoimmunity (Rieux-Laucat, et al. (1995) *Science* 268, 1347-1349; and Fischer, G.H. (1995) *Cell* 81, 935-946). Ligation of the cell surface Fas molecule rapidly and dramatically induces apoptosis in many but not all Fas positive cell types (Nagata, S. (1997) *Cell* 88, 355-365). DT-40 is a chicken lymphoma B-cell line that has been used to elucidate the molecular mechanism of radiation-induced apoptosis (Uckun, F.M., et al. (1996) *Science* 273, 1096-1100). Despite their abundant surface expression of Fas, DT-40 cells, similar to human B-cell precursor leukemia cells, are resistant to the cytotoxic effects of Fas-ligation, indicating the existence of potent negative regulators of Fas-mediated apoptosis. Bruton's tyrosine kinase (BTK), a member of the BTK/Tec family of protein tyrosine kinases (PTKs) is a cytoplasmic PTK involved in signal transduction pathways regulating growth and differentiation of B-lineage lymphoid cells (Rawlings, D. J., and Witte, O. N. (1994) *Immunol. Rev.* 138,105-119; Kurosaki, T. (1997) *Curr Opin. Immunol.* 9, 309-318; and Uckun, F. M. (1998) *Biochemical Pharmacology,* et al., 56, 683-691). BTK participates in signal transduction pathways initiated by the binding of a variety of extracellular ligands to their cell surface receptors: following ligation of B cell antigen receptors (BCR), BTK activation by the concerted actions of the PTKs Lyn and Syk (Kurosaki, T. (1997) *Curr Opin. Immunol.* 9, 309-318) is required for induction of phospholipase C-γ2 mediated calcium mobilization (Kurosaki, T. (1997) *Curr Opin. Immunol.* 9, 309-318). Mutations in the human *BTK* gene are the cause of X-linked agammaglobulinemia (XLA), a male immune deficiency disorder characterized by a lack of mature, immunoglobulin producing, peripheral B cells (Tsukada, S., et al. (1993) *Cell* 72, 279-290; and Vetrie, D., et al. (1993) *Nature* 361, 226-233). In mice, mutations in the *BTK* gene have been identified as the cause of murine X-linked immune deficiency (Xid) (Rawlings, D. J., et al. (1993) *Science* 261, 358-361).

BTK has been shown to be an inhibitor of the Fas/APO-1 death inducing signaling complex (DISC) in B-lineage lymphoid cells (Vassilev, A., et al. (1998) *J*. *Biol. Chem., 274,* 1646-1656). Additionally, it has presently been determined that BTK prevents ceramide- and vincristine-induced apoptosis (present study). The fate of leukemia/lymphoma cells may reside in the balance between the opposing proapoptotic effects of caspases activated by DISC and an upstream anti-apoptotic regulatory mechanism involving BTK and/or its substrates (Vassilev, A., et al. (1998) *J. Biol. Chem.,* 274, 1646-1656). Inhibitors of BTK are likely to enhance the drug sensitivity of B-lineage (e.g. leukemia/lymphoma) cells. Thus, pharmacological agents with BTK-modulatory activity can be used as chemosensitizing agents for treating BTK-expressing malignancies or diseases caused by proliferation and antibody production of BTK-expressing B-cells, and as B-cell reconstituting agents in humoral immunodeficiencies with decreased numbers or absence of B-cells. Further BTK modulating agents would be useful as immunosuppressive agents for prevention of hyperacute rejection of organs in transplantation, which is directed by B-cells, autoimmune diseases, and conversion of immunity to drugs (e.g. antibodies or biologicals) or blood products (e.g. coagulation factors such as Factor VIII) in patients who develop antibodies to such agents.

### Identification of inhibitors of BTK

The potent and selective BTK inhibitor LFM-13 and other BTK inhibitors were identified using the three-dimensional homology model of the kinase domain described in Example 2. Using this model and the size and contact information provided in Examples 1 and 3, additional BTK inhibitors were designed and tested. Using this model and method, other compounds that interact favorably with the binding pocket can be identified, as well as compounds that will bind selectively to BTK over other related kinases. Tight binding or a good fit in the binding pocket model correlates with potent BTK-inhibitory activity.

The ability of an agent to inhibit the anti-apoptotic effects of BTK can be measured using assays that are known in the art, or using the assays disclosed in the Examples hereinbelow. Thus, using the modeling information and the screens described herein, as well as other information known in the art, one can identify agents that possess BTK inhibiting properties.

Inhibitors of BTK also include those inhibitors produced by recombinant DNA methods, such as antisense molecules and transcription inhibitors. Initial studies on *btk* transcription have demonstrated that expression of the *btk* gene is regulated by the combined action of Sp1-and PU.1-family transcription factors (S. Muller et al. *Oncogene,* 1996, *13,* 1955-1964; and A. Himmelmann et al. *Blood,* 1996, *87*, 1036-1044). Transcriptional regulatory elements have been identified within the first and tenth introns of the *btk* gene, and recent studies indicate that regulation of *btk* gene expression involves multiple transcription factors ( J. Rohrer, M.E. Conley, *Blood,* 1998, *91,* 214-221). New agents affecting the activity of these transcription factors would also be useful as modulators of apoptotic signals in treatment programs. The feasibility of regulating *btk* gene expression in human hematopoietic cells has been already been demonstrated by the ability of retinoic acid to increase *btk* expression in myeloid cells, and by the ability of phorbol ester as well as TGF- 1 to decrease *btk* expression in B-cells (C.I.E. Smith et al., *J. Immunol,* 1994, *152*, 557-565).

Thus, one or more of the recombinant DNA methods can be used to inhibit BTK expression and induce the therapeutic effects discussed herein. Such methods include, for example, antisense sequences of transcription inhibitors. For example, BTK antisense constructs may be directed against BTK expression directly. Alternatively, the antisense construct may be directed against BTK regulatory sequences, e.g., antisense sequences to the Sp1 and PU.1 family of transcription factors. Preferably, the antisense constructs are targeted to tumor cells.

### Compounds:

Compounds of formula I are specific BTK inhibitors that bind favorably to the BTK model pocket described in the examples, and have potent **BTK** inhibitory activity.

The compounds of formula I are: 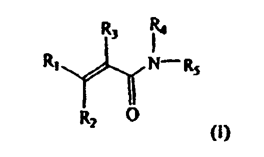 where:
R₁ is (C₁-C₃)alkyl, (C₃-C₆)cycloalkyl, phenyl, or NRₐR_{b};
R₂ is hydroxy, (C₁-C₆)alkoxy, (C₁-C₆)alkanoyloxy, amino (C₂-C₅)alkoxy; hydroxy (C₂-C₅)alkoxy, amino (C₂-C₅)alkanoyloxy; or hydroxy (C₁-C₅) alkanoyloxy;
R₃ is cyano or (C₁-C₃)alkanoyl;
R₄ is hydrogen, (C₁-C₃)alkyl; hydroxy (C₂-C₅)alkyl; or amino (C₂-C₅)alkyl;
R₅ is phenyl substituted by -S(O)₂ Rc, or by halo and at least one other substituent;
Rₐ and R_{b} are each independently hydrogen, or (C₁-C₃)alkyl; or Rₐ and R_{b} together with the nitrogen to which they are attached are pyrrolidino, piperidino, morpholino, or thiomorpholino;
wherein any aryl, or heteroaryl of R₁ and R₅ is optionally substituted with one or more (e.g. 1, 2, or 3) substituents independently selected from halo, nitro, cyano, hydroxy, trifluoromethyl, trifluoromethoxy, (C₁-C₃)alkoxy, (C₁-C₃)alkyl, (C₁-C₃)alkanoy), -S(O)₂R_{c}, or NRₐR_{b}; wherein R_{c} is (C₁-C₃)alkyl, or aryl
or a pharmaceutically acceptable salt thereof.

Particularly useful compounds of formula I include:
α-Cyano-β-hydroxy-β-methyl-*N*-(2,5-dibromophenyl)-propenamide;
α-Cyano-β-hydroxy-β-methyl-*N*-[4-(methylsulfonyl)phenyl]-propenamide;
α-Cyano-β-hydroxy-β-methyl-*N*-[3-(methylsulfonyl)phenyl]-propenamide;
α-Cyano-β-hydroxy-β-methyl-*N*-[3-bromo-4-(trifluoromethoxy)-phenyl]propenamide;
α-Cyano-β-hydroxy-β-methyl-*N*-(2,4-dibromophenyl)-propenamide;
α-Cyano-β-hydroxy-β-methyl-*N*-(2,4-dichlorophenyl)-propenamide;
α-Cyano-β-hydroxy-β-methyl-*N*-(2,5-dichlorophenyl)-propenamide; or
α-Cyano-β-hydroxy-β-methyl-*N*-(3,4-dichlorophenyl)-propenamide; or
pharmaceutically aceptable salts thereof.

### Definitions:

The following definitions are used herein, unless otherwise described: halo is fluoro, chloro, bromo, or iodo. Alkyl, alkoxy, etc. denote both straight and branched groups; but reference to an individual isomer such as "propyl" embraces only the straight chain isomer, a branched chain isomer such as "isopropyl" being specifically referred to. Aryl denotes a phenyl group or a bicyclic or tri-cyclic carbocyclic group having about nine to twelve ring atoms in which at least one ring is aromatic.

It will be appreciated by those skilled in the art that compounds of Formula I having a chiral center may exist in and be isolated in optically active and racemic forms. Some compounds may exhibit polymorphism. It is to be understood that the present invention encompasses the use of any racemic, optically-active, polymorphic, or stereoisomeric form, or mixtures thereof, of a compound of Formula I, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine BTK inhibiting activity using the standard assays described herein, or using other similar assays which are well known in the art.

Specific and preferred values listed below for substituents and ranges, are for illustration only; they do not exclude other defined values or other values within defined ranges for the radicals and substituents

Specifically, (C₁-C₃)alkyl can be methyl, ethyl, propyl, or isopropyl; (C₃-C₆)cycloalkyl can be cyclopropyl, cyclobutyl, cyclopentyl, or cyclohexyl (C₁-C₃)alkoxy can be methoxy, ethoxy, propoxy, isopropoxy; (C₁-C₆)alkoxy can be methoxy, ethoxy, propoxy, isopropoxy, butoxy, iso-butoxy, sec-butoxy, pentoxy, 3-pentoxy, or hexyloxy;

### Specific and preferred values

A specific value for R₁ is (C₁-C₃)alkyl, or (C₃-C₆)cycloalkyl.

A specific value for R₂ is hydroxy.

A specific value for R₃ is cyano.

A specific value for R₄ is hydrogen.

A specific value for R₅ is phenyl substituted with halo, and substituted with 1, 2, or 3 other substituents independently selected from halo, nitro, cyano, trifluoromethyl, trifluoromethoxy, (C₁-C₃)alkoxy, (C₁-C₃)alkyl, (C₁-C₃)alkanoyl and NRₐR_{b}.

A more specific value for R₁ is (C₁-C₃)alkyl.

A more specific value for R₅ is phenyl substituted with halo, and substituted with 1, 2, or 3 other substituents independently selected from halo, trifluoromethyl, trifluoromethoxy, and (C₁-C₃)alkoxy.

A more specific value for R₅ is phenyl substituted with 2 or 3 halo.

A more specific value for R₅ is phenyl substituted with two bromo.

A preferred compound of formula I is α-cyano-β-hydroxy-β-methyl-*N*-(2,5-dibromophenyl)propenamide; or a pharmaceutically acceptable salt thereof.

### Therapeutic Use

B-cells and B-cell precursors expressing BTK have been implicated in the pathology of a number of diseases and conditions including B-cell malignancies (e.g. acute lymphoblastic leukemia, chronic lymphocitic leukemia, non-Hodgkin's lymphoma, EBV lymphomia, and myeloma), other cancers, B-cell lymphoproliferative disorders/autoimmune diseases (e.g. lupus, Crohn's disease, and chronic or graft-versus-host disease), mast cell disorders (e.g. allergies, and anaphylactic shock), conditions that relate to improper platelet aggregation, and rejection of xenotransplants (e.g. pig to human heart transplants).

Additionally, the selective BTK inhibitors can be used to identify other diseases wherein BTK plays a role, and particularly to identify gene expression that is modulated by BTK. This can be done using techniques that are known in the art, for example, using gene profiling techniques similar to those described by A. Sehgal et al., *Journal of Surgical Oncology,* 1998, *67*, 234-241. Incubating cells in the presence or absence of a BTK inhibitor followed by profiling of gene expression in the cells is useful to identify BTK-regulated gene expression. Materials useful for profiling gene expression using Atlas cDNA membranes can be obtained from CLONTECH Laboratories, Inc. 1020 East Meadow Circle, Palo Alto, CA 94303. cDNA microarrays can also be ordered from commercial sources or be custom made.

Using such materials according to the manufacturer's instructions, it has also been discovered that BTK modulates the expression of specific genes, for example, MAPKAP kinase and c-myc oncogene. This activity suggests that BTK may be implicated in the pathology of all forms of cancer.

BTK is a member of the Tec family of tyrosine kinases. Tec kinase is expressed, for example, in T-cells. The BTK inhibitors of the invention are also useful to inhibit the activity of other members of the Tec kinase family.

BTK inhibitors (including compounds of formula I as described herein) can be used to treat disorders wherein the inhibition or prevention of a Tec family kinase, including BTK activity is indicated. It has also been discovered that BTK inhibitors are useful as chemosensitizing agents, and, thus, useful in combination with other chemotherapeutic drugs, in particular, drugs that induce apoptosis. Examples of other chemotherapeutic drugs that can be used in combination with chemosensitizing BTK inhibitors include topoisomerase I inhibitors (camptothesin or topotecan), topoisomerase II inhibitors (e.g. daunomycin and etoposide), alkylating agents (e.g. cyclophosphamide, melphalan and BCNU), tubulin directed agents (e.g. taxol and vinblastine), and biological agents (e.g. antibodies such as anti CD20 antibody, IDEC 8, immunotoxins, and cytokines).

### Conjugation to a Targeting Moiety

The compounds of formula I can be targeted for specific delivery to a cell type to be treated by conjugation of the BTK inhibitor to a targeting moiety. Targeting moieties useful for conjugation to BTK inhibitors include antibodies, cytokines, and receptor ligands that are specific to the cell to be treated.

The term "conjugate" means a compound formed as a composite between two or more molecules. More specifically, in the present invention, the quinazoline derivative is bonded, for example, covalently bonded, to cell-specific targeting moieties forming a conjugate compound for efficient and specific delivery of the agent to a cell of interest.

The phrase "targeting moiety" means a molecule which serves to deliver the compound of the invention to a specific site for the desired activity. Targeting moieties include, for example, molecules that specifically bind molecules on a specific cell surface. Such targeting moieties useful in the invention include anti-cell surface antigen antibodies. Cytokines, including interleukins and factors such as granulocyte/macrophage stimulating factor (GMCSF) are also specific targeting moieties, known to bind to specific cells expressing high levels of their receptors.

Particularly useful targeting moieties for targeting the BTK-inhibitory compounds of the invention to cells for therapeutic activity include those ligands present of Tec kinase expressing cells. For example, antigens present on B-cells and B-lineage cancer cells, such as CD19 can be targeted with anti-CD 19 anitbodies such as B43. Antibody fragments, including single chain fragments can be used. Natural ligands for the surface antigens such as CD19 can also be used. Tec kinase expressing T cells can be targeted, for example to the CD7 antigen with anti-CD7 antibodies such as TXU. Mast cells can be targeted via the CD48 antigen with anti-CD48 antibodies. These and other cell surface antigen antibodies are commercially available, for example, from Pharmingen.

Cytokines are also useful targeting moieties. T cells can be targeted with IL2 and IL7; B cells can be targeted with IL4; mast cells can be targeted with C-KIT, MGF, GMCSF, and IL3. Cancer cells expressing Tec kinase can be targeted, for example, with EGF and IGF.

### Compounds as Salts

In cases where an agent ("compound") is sufficiently basic or acidic to form stable nontoxic acid or base salts, administration of the compound as a salt may be appropriate. Examples of pharmaceutically acceptable salts are organic acid addition salts formed with acids which form a physiologically acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

### Prodrug Derivatives

The compounds of formula I may have attached thereto functional groups to provide a prodrug derivative. The prodrug derivative facilitates use of the drug in the body, for example, by facilitating entry into cells. The term "prodrug moiety" is a substitution group that facilitates use of a compound of the invention, for example by facilitating entry of the drug into cells or administration of the compound. The prodrug moiety may be cleaved from the compound, for example by cleavage enzymes *in vivo.* Examples of prodrug moieties include phosphate groups, peptide linkers, and sugars, which moieties can be hydrolyzed *in vivo.*

### Pharmaceutical formulations

A compound can be formulated as pharmaceutical compositions and administered to a mammalian host, such as a human patient in a variety of forms adapted to the chosen route of administration, i.e., orally or parenterally, by intravenous; intramuscular, topical, or subcutaneous routes.

Thus, compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Any material used in preparing any unit dosage form should be pharmaceutically acceptable and substantially non-toxic in the amounts employed. In addition, the active compound may be incorporated into sustained-release preparations and devices.

The compound may also be administered intravenously or intraperitoneally by infusion or injection. Solutions of the compound or its salt can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active compound which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions can be prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active compound plus any additional desired ingredient present in the previously sterile-filtered solutions.

For topical administration, the compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions that can be used to deliver the compounds of the invention to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compounds of the invention can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the compound(s) of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from about 0.5 to about 100 mg/kg, e.g., from about 10 to about 75 mg/kg of body weight per day, such as 3 to about 50 mg per kilogram body weight of the recipient per day, preferably in the range of 6 to 90 mg/kg/day, most preferably in the range of 15 to 60 mg/kg/day.

The compound is conveniently administered in unit dosage form; for example, containing 5 to 1000 mg, conveniently 10 to 750 mg, most conveniently, 50 to 500 mg of active compound per unit dosage form.

Ideally, the active compound should be administered to achieve peak plasma concentrations of the active compound of from about 0.5 to about 75 µM, preferably, about 1 to 50 µM, most preferably, about 2 to about 30 µM. This may be achieved, for example, by the intravenous injection of a 0.05 to 5% solution of the active ingredient, optionally in saline, or orally administered as a bolus containing about 1-100 mg of the active ingredient. Desirable blood levels may be maintained by continuous infusion to provide about 0.01-5.0 mg/kg/hr or by intermittent infusions containing about 0.4-15 mg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

As disclosed herein, it has been discovered that BTK inhibitors are useful as chemosensitizing agents, and thus, are useful to increase the sensitivity of a cancer cell to other chemotherapeutic agents that promote apoptosis. As such, BTK inhibitors can conveniently be administered in combination with other chemotherapeutic agents. Additionally, the pharmaceutical compositions that comprise an agent that inhibits BTK, can also further comprise one or more other chemotherapeutic agents that promote apoptosis.

The invention will now be illustrated by the following non-limiting Examples.

### EXAMPLES

### Example 1 BTK inhibits FAS/APO-1 DISC

The following example provides biochemical and genetic evidence that BTK is an inhibitor the Fas/APO-1 death inducing signaling complex (DISC) in B-lineage lymphoid cells. BTK associates with Fas via its kinase and PH domains and prevents the FAS-FADD interaction, which is essential for the recruitment and activation of the death-inducing proteolytic enzyme FLICE by Fas during the apoptotic signal. Notably, treatment of human leukemic B-cells with a potent inhibitor of BTK abrogated the BTK-Fas association and sensitized the cells to Fas-mediated apoptosis.

**Cell lines, reagents, and biochemical assays.** The establishment of BTK-deficient DT-40 lymphoma B-cell clones has been previously described by Uckun, F.M., et al. (1996) *Science* 273, 1096-1100. To disrupt the *btk* gene, targeting constructs containing neomycin-resistance gene cassette (i.e., pcBTK-neo) or histidinol-resistance gene cassette (i.e., pcBTK-hisD) were sequentially transfected into DT-40 cells. The targeting vectors, pcBTK-neo and pcBTK-hisD, were constructed by replacing the 0.7 kb *Bgl*II*-Bam*HI genomic fragment containing exons which correspond to human BTK amino acid residues 91-124 with *neo* or *hisD* cassette. pcBTK-neo was linearized and introduced into wild-type DT-40 cells by electroporation. Screening was done by Southern blot analysis using a 3' flanking probe (0.5 kb BgllI-Bgl-II fragment). The neo-targeted clone was again transfected with pcBTK-hisD and selected with both G418 (2 mg/mL) and histidinol (1 mg/mL). Southern blot analysis of BTK-deficient DT-40 clone confirmed the homologous recombination at both *btk* loci and hybridization with a neo and hisD probe indicated that the targeted clone had incorporated a single copy of each construct. Lack of BTK expression in BTK-deficient DT-40 cells was confirmed by both immune complex kinase assays and Western blot analysis (Uckun, F.M., et al. (1996) *Science* 273, 1096-1100). Mutations in the human *btk* cDNA were introduced by PCR using Pfu polymerase (Strategene, La Jolla, California, #600153) and confirmed by sequencing. Wild-type and mutant *btk* cDNAs were subcloned into pApuro expression vector and electroporated into BTK-deficient cells. The PTK activity of BTK immune complexes, as measured by in vitro autophosphorylation, was abrogated by the catalytic domain mutation, reduced by the PH domain mutation but not affected by the mutation in the SH2 domain. Equal amounts of BTK protein were detected by Western blot analysis in all of the BTK-deficient DT-40 clones transfected with wild-type or mutated human *btk* genes but no BTK protein was detectable in the untransfected BTK-deficient DT-40 cells (Uckun, F.M., et al. (1996) *Science* 273, 1096-1100). The establishment and characterization of LYN-deficient DT-40 clones were previously reported by Uckun, F.M., et al. (1996) *Science* 273,1096-1100. In addition to these chicken lymphoma B-cells, the following human B-lineage lymphoid cell lines: NALM-6-UM1, BTK-positive human B-cell precursor (pre-B acute lymphoblastic leukemia) cell line; RAMOS-1, BTK-deficient human Burkitt's/B-cell leukemia line; and KL2 BTK-positive human EBV-transformed normal B-lymphoblastoid cell lines were used.

Antibodies directed against BTK, SYK, and LYN have been described previously (Uckun, F.M., et al. (1996) *Science* 273, 1096-1100; Dibirdik, I., et al. (1998) *J. Biol. Chem.,* 273, 4035-4039; and Uckun, F.M., et al. (1996) *J. Biol. Chem.* 271, 6389-6397. Polyclonal antibodies to BTK were generated by immunization of rabbits with glutathione S-transferase (GST) fusion proteins (Amersham Pharmacia Biotech, Arlington Heights, Illinois) containing the first 150 amino acids of BTK. In addition, the following anti-BTK antibodies were used in Western blots of purified fusion proteins: Polyclonal goat anti-BTK carboxyl terminus (Santa Cruz Biotechnology, Inc., Santa Cruz, Ca., cat # SC1107), polyclonal goat anti-BTK amino terminus (Santa Cruz Biotechnology, Inc., Santa Cruz, Ca. cat # SC 1108,), and polyclonal rabbit serum raised against the Btk SH₂-SH₃ domains (aa219-377). Polyclonal anti-MBP antibodies were generated by immunizing rabbits. The rabbit polyclonal anti-Fas (sc-715 mixed 1:1 with sc-714) which crossreacts with both human and chicken Fas proteins, goat polyclonal anti-FADD (sc-1171), goat polyclonal anti-TRADD (sc-1163), goat polyclonal anti-FLICE (sc-6135) were purchased from Santa Cruz Biotechnology, Inc. and used according to the manufacturer's recommendations. The monoclonal anti-Fas antibody (F22120) was obtained from the Transduction Laboratories, Inc. (Lexington, KY, USA). Immunoprecipitations, immune-complex protein kinase assays, and immunoblotting using the enhanced chemiluminescence (ECL) detection system (Amersham Pharmacia Biotech, Arlington Heights, Illinois) were conducted as described previously (Uckun, F.M., et al. (1996) *Science* 273, 1096-1100; Dibirdik, I., et al. (1998) *J. Biol. Chem.,* 273, 4035-4039; Uckun, F.M., et al. (1996) *J. Biol. Chem.* 271, 6389-6397; Mahajan, S., et al. (1995) *Mol. Cell. Biol.* 15, 5304-5311; Uckun, F.M., et al. (1993) *J. Biol. Chem.* 268, 21172-21184; Uckun, F.M., et al. (1995) *Science* 267, 886-91; and Uckun, F.M., et al. (1997) *Blood,* 89, 3769-3777). The BTK inhibitor α-cyano-β-hydroxy-β-methyl-*N*-(2,5-dibromophenyl)propenamide (LMA-13) was prepared as described in Example 2.

**Expression and Purification of MBP-BTK and GST-BTK Fusion Proteins.** cDNAs encoding full-length BTK and its kinase or PH domains with PCR-generated 5' and 3' BamHI sites were cloned into the E. coli expression vector pMAL-C2 with the IPTG-inducible Ptac promoter to create an in frame fusion between these coding sequences and the 3' end of the E. coli malE gene, which codes for maltose binding protein (MBP). cDNAs encoding the SH2, SH3, or SH2+SH3 domains with PCR-generated 5' and 3' BamHI sites were cloned into the E. coli expression vector pGEX-2t with the IPTG-inducible Ptac promoter to create an in frame fusion between these coding sequences and the 3' end of the E. coli gluthatione S-transferase (GST) gene. The generated recombinant plasmids were transformed into the E. coli strain DH5a. Single transformants were expanded in 5 ml Luria-Burtain (LB) medium (1% tryptone, 1% NaCl, 0.5% yeast extract) containing ampicillin (100 µg/ml) by overnight culture at 37°C. Expression of the fusion proteins was induced with 10 mM IPTG. The cells were harvested by centrifugation at 4,500g in a Sorvall RC5B centrifuge for 10 minutes at 4°C, lysed in sucrose-lysozyme buffer (20 mM Tris pH 8.0, 150 mM Nacl, 10 % sucrose, 1 mM EDTA, 20 mM lysozyme), and further disrupted by sonication. After removal of the cell pellets by centrifugation at 35,000g for 1 hour at 4 °C, GST-BTK fusion proteins were purified by gluthatione-Sepharose chromatography (Uckun, F.M., et al. (1996) *J. Biol. Chem.* 271, 6389-6397), whereas MBP-BTK fusion proteins were purified from the culture supernatants by amylose affinity chromatography (Hsu, D., et al. (1996) *Biochemistry* 35, 13871-77).

**Confocal Laser Scanning Microscopy.** Wildtype and BTK-deficient DT-40 cells treated with anti-Fas antibody (1 µg/ml, 24 hrs at 37°C) were attached to poly-1-lysine coated coverslips and fixed in ice cold (-20°C) methanol for 15 minutes. After fixation, the coverslips were washed for 15 min in phosphate buffered saline (PBS) + 0.1% triton X-100. Cells were stained with a rabbit polyclonal anti-tubulin antibody according to the manufacturer's recommendations (Sigma, St. Louis, MO) to visualize their cytoplasms. DNA was labeled for 10 min with toto-3 a DNA specific dye (Molecular Probes, Eugene OR) to visualize the apoptotic changes in the nuclei. BTK-MBP electroporated BTK-deficient DT-40 cells and non-electroporated BTK-deficient DT-40 cells were labeled with an antibody raised against maltose binding protein. The secondary antibody was a goat anti-rabbit fluorescein conjugated antibody. In some experiments, cells were examined for Fas expression by confocal microscopy. In brief, cells were attached to poly-L-lysine coated coverslips and fixed for 40 min in 2% paraformaldehyde in phosphate buffered saline (PBS). Cells were rinsed in PBS + 115 mM glycine to quench the formaldehyde and then blocked in PBS containing 2% bovine serum albumin (PBS+BSA). A monoclonal antibody raised against the extracellular domain of Fas (Transduction Labs, Lexington, KY, catalog #F22120) was added in PBS+BSA and the coverslips were incubated for 40 min at 37° C before again rinsing in PBS. A fluorescein-labeled secondary antibody (goat antimouse - FITC H+L, Zymed, South San Francisco, CA , catalog no. 62-6511) diluted in PBS+BSA was then added to the coverslips and they were again incubated for 40 min at 37° C. After another wash, cellular DNA was labeled by incubation in 1 mM TOTO-3 (Molecular Probes, Eugene, OR) for 20 min at room temperature. Coverslips were inverted and mounted onto slides in Vectashield (Vector Labs, Burlinghame, CA), to prevent photobleaching and sealed with nail varnish. Slides were examined using a Bio-Rad MRC 1024 Laser Scanning Confocal Microscope mounted on an Nikon Eclipse E-800 upright microscope equipped for epifluorescence with high numerical aperture objectives (Uckun, F.M., et al. (1998) *Clinical Cancer Research,* 4, 901-912). Optical sections were obtained and turned into stereo micrographs using Lasersharp software (Bio-Rad, Hercules, CA). Representative digital images were saved to Jaz disk and processed using the Photoshop software (Adobe Systems, Mountain View CA). Images were printed with a Fuji Pictography thermal transfer printer (Fuji Photo, Elmsford, NY). Digital data were archived and stored on CD-ROM.

**Apoptosis Assays.** To induce apoptosis, cells were treated with an agonistic anti-Fas/APO-1 antibody (Biosource International, Camarillo, Ca., lot. 04/1295) at 0.1 µg/ml, 0.5 µg/ml, or 1.0 µg/ml final concentrations. MC540 binding (as an early marker of apoptosis) and PI permeability (as a marker of advanced stage apoptosis) were simultaneously measured in DT-40 cells 24 hours after exposure to anti-Fas antibody as previously described (Uckun, F.M., et al. (1996) *Science 273,* 1096-1100). Whole cells were analyzed with a FACStar Plus flow cytometer (Becton Dickinson, San Jose, CA). All analyses were done using 488 nm excitation from an argon laser. MC540 and PI emissions were split with a 600 nm short pass dichroic mirror and a 575 nm band pass filter was placed in front of one photomultiplier tube to measure MC540 emission and a 635 nm band pass filter was used for PI emission.

To detect apoptotic fragmentation of DNA, DT-40, NALM-6-UM1, and RAMOS-1 cells were harvested 24 hours after exposure to anti-Fas. DNA was prepared from Triton-X-100 lysates for analysis of fragmentation (Uckun, F.M., et al. (1996) *Science* 273, 1096-1100; Uckun, F.M., et al. (1995) *Science* 267, 886-91; and Uckun, F.M., et al. (1998) *Clinical Cancer Research,* 4, 901-912°). Cells were lysed in hypotonic 10 mmol/L Tris-HCl (pH 7.4), 1 mmol/L EDTA, 0.2% Triton-X-100 detergent; and subsequently centrifuged at 11,000g. To detect apoptosis-associated DNA fragmentation, supernatants were electrophoresed on a 1.2% agarose gel, and the DNA fragments were visualized by ultraviolet light after staining with ethidium bromide. In some experiments, MBP-BTK fusion proteins (100 µg/2.5x10⁸ cells) were electroporated (420V electrical field, 125 µF) into BTK-deficient DT-40 cells using a BioRad gene pulser and the procedures of Bergland and Starkey (Bergland, D. and Starkey, J. (1991) *Cytometry* 12, 64-67) with slight modifications 4 hours prior to Fas-ligation and apoptosis assays.

**Pull Down Assays with MBP-BTK and GST-BTK Fusion Proteins.** GST-BTK fusion proteins were non-covalently bound to glutathione-agarose beads (Sigma Aldrich, Inc., St. Louis, MO) and MBP-BTK fusion proteins were non-covalently bound to amylose beads under conditions of saturating protein, as previously described (Uckun, F.M., et al. (1996) *J. Biol. Chem.* 271, 6389-6397; and Hsu, D., et al. (1996) *Biochemistry* 35, 13871-77). In brief, 50 µg of each protein was incubated with 50 µl of the beads for 2 hours at 4°C. The beads were washed three times with 1% Nonidet P-40 buffer. Nonidet P-40 lysates of BTK-deficient DT-40 cells, NALM-6-UM1 human leukemic B-cell precursors, and KL2 human EBV-transformed lymphoblastoid cells were prepared as described (Uckun, F.M., et al. (1996) *Science* 273, 1096-1100; and Uckun, F.M., et al. (1996)*J*. *Biol. Chem.* 271, 6389-6397) and 500 µg of the lysate was incubated with 50 µl of fusion-protein coupled beads for 2 hours on ice. The fusion protein adsorbates were washed with ice-cold 1% Nonidet P-40 buffer and resuspended in reducing SDS sample buffer. Samples were boiled for 5 min and then fractionated on SDS-PAGE. Proteins were transferred to Immobilon-P (Millipore, Bedford, MA) membranes, and membranes were immunoblotted with anti-Fas (Transduction Laboratories, Inc., Lexington, KY, cat. # F22120), according to previously described procedures (Uckun, F.M., et al. (1996) *Science* 273, 1096-1100; Dibirdik, I., et al. (1998) *J*. *Biol. Chem.,* 273, 4035-4039; Uckun, F.M., et al. (1996) *J. Biol. Chem.* 271, 6389-6397; and Uckun, F.M., et al. (1997) *Blood,* 89, 3769-3777).

In a series of experiments designed to examine the potential negative regulatory role of BTK in Fas-mediated apoptosis, we first compared the effects of Fas-ligation on wild-type DT-40 cells to the effects of Fas-ligation on a BTK-deficient subclone of DT-40 cells that was established by homologous recombination knockout (Uckun, F.M., et al. (1996) *Science* 273, 1096-1100). To this end, we first used a quantitative flow cytometric apoptosis detection assay (Uckun, F.M., et al. (1996) *Science* 273, 1096-1100). MC540 binding and propidium iodide (PI) permeability were simultaneously measured before and after treatment with the agonistic anti-Fas antibody (1 µg/mL x 24 hours). Whereas only 5.0% of wild-type DT-40 cells treated with the anti-Fas antibody showed apoptotic changes, 96.3% of BTK-deficient DT-40 cells underwent apoptosis, as determined by MC540 single fluorescence (early apoptosis) or MC540/PI double fluorescence (advanced apoptosis) at 24 hours (Figure 1). Notably, BTK-deficient DT-40 cells reconstituted with a wild-type human *btk* gene displayed very little flow cytometric evidence of apoptosis, which provided formal evidence that BTK plays a pivotal role in preventing the apoptotic death signal triggered by Fas-ligation. In accordance with previously published information regarding the pro-apoptotic function of Src family PTK (Waddick, K.G., et al. (1993) *Radiation Research,* 136, 313-319; Schlottmann, K.E., et al. *Leukocyte Biology* 60, 546-554; and Atkinson, E.A., et al. (1996) *J. Biol. Chem.* 271, 5968-5971) and the reported impairment of Fas-mediated apoptosis in B-cells from LYN-deficient mice (Wang, J., et al. (1996) *J. Exp. Med.* 184, 831-838), very little apoptosis was found in an anti-Fas treated LYN-deficient subclone of DT-40 cells that was included as a control in these experiments (Figure 1). As shown in Figure 2A, no BTK protein was detectable by Western blot analysis in the whole cell lysates of BTK-deficient DT-40 cells, whereas BTK-deficient DT-40 cells reconstituted with a wild-type human *btk* gene expressed higher levels of BTK than the wild-type DT-40 cells. However, the Fas protein expression levels in these three B-cell clones were virtually identical (Figure 2B). These findings were further confirmed by confocal microscopy. As shown in C.1 to C.3, all three cell lines exhibited similar levels of punctate Fas staining. 3-dimensional reconstructions of serial optical sections confirmed the expression of Fas both in the cytoplasm and on the surface membrane of all three cell lines without any detectable differences relative to expression levels or pattern. Thus, the resistance of wild-type DT-40 cells or BTK-deficient DT-40 cells reconstituted with wild-type BTK against Fas-mediated apoptosis was not due to lower expression levels of Fas protein and the susceptibility of BTK-deficient DT-40 cells to Fas-mediated apoptosis was not caused by augmented Fas protein expression.

The comparative examination of the morphologic features of wild-type versus BTK-deficient DT-40 cells by laser scanning confocal microscopy showed no evidence of apoptosis for wild-type cells after treatment with the agonistic anti-Fas antibody, whereas BTK-deficient cells showed shrinkage and nuclear fragmentation consistent with apoptosis (Figure 3A). On agarose gels, DNA from Triton-X-100 lysates of anti-Fas treated BTK-deficient DT-40 cells showed a ladder-like fragmentation pattern consistent with apoptosis, whereas no DNA fragmentation was observed in wild-type DT-40 cells (Figure 3B). These results provide direct evidence that BTK can inhibit Fas/APO-1-mediated apoptosis.

BTK has a unique N-terminal region which contains a pleckstrin homology (PH) and Tec homology (TH) domains, a single Src homology 3 (SH3) domain which contains the autophosphorylation site at tyrosine 223, a single SH2 domain, and a catalytic kinase domain which contains the transphosphorylation site at tyrosine 551 (Rawlings, D.J., Witte, O.N. (1994) *Immunol. Rev.* 138, 105-119; and Kurosaki, T. (1997) *Curr Opin. Immunol.* 9, 309-318). The PH domain of BTK interacts with various isoforms of protein kinase C, heterotrimeric G proteins, as well as the BAP-135 protein (Rawlings, D.J., Witte, O.N. (1994) *Immunol. Rev.* 138, 105-119; Kurosaki, T. (1997) *Curr Opin. Immunol.* 9, 309-318; Tsukada, S. (1994) *J. Biol. Chem.* 269, 10217-10220; and Yang, W., Desiderio, S. (1997) *Proc Natl Acad Sci USA* 94, 604-609). SH3 domains have been shown to interact with proline-rich sequences of other proteins whereas SH2 domains interact with tyrosine phosphorylated proteins (Yang, W., Desiderio, S. (1997) *Proc Natl Acad Sci USA* 94, 604-609). However, specific proteins interacting with the BTK SH2 or SH3 domains in B-lineage lymphoid cells have not been reported. Mutations in the catalytic domain, SH2 domain, as well as PH domain of the BTK have been found to lead to maturational blocks at early stages of B-cell ontogeny in human XLA (Pawson,T., Gish, G.D. (1992) *Cell* 71, 359-362; and Vihinen, M., et al. (1995) *Immunol. Today* 16, 460-465). BTK-deficient mice generated by introducing PH-domain or catalytic domain mutations in embryonic stem cells showed defective B-cell development and function (Kerner, J.D., et al. (1995) *Immunity* 3, 301-312. Thus, different regions of BTK are important for its physiologic functions. To examine the participation of the various domains of BTK in negative regulation of Fas-mediated apoptosis, we introduced wild-type human *btk* gene as well as human *btk* genes harboring mutations either in the catalytic domain (Arg⁵²⁵ to Gln), SH2 domain (Arg³⁰⁷ to Ala), or PH domain (Arg²⁸ to Cys) into the BTK-deficient DT-40 cells (Uckun, F.M., et al. (1996) *Science* 273, 1096-1100). As evidenced in Figure 3C & 3D, BTK-deficient DT-40 cells reconstituted with wild-type human *btk* gene (rWT) did not undergo apoptosis after treatment with the agonistic anti-Fas antibody, whereas Fas-activation of reconstituted BTK-deficient DT-40 cells expressing human BTK with mutations in the kinase (rK-), SH2 (rmSH2), or PH (rmPH) domains induced apoptosis as it did in non-reconstituted BTK-deficient DT-40 cells shown in Figure 3B. Thus, the kinase, SH2, and PH domains of BTK are all important and apparently indispensable for its function as a negative regulator of Fas-mediated apoptosis.

To further characterize the anti-apoptotic function of BTK, we introduced by electroporation an MBP fusion protein containing full-length wild-type BTK into BTK-deficient cells 4 hours prior to treatment with the anti-Fas antibody. Examination of these cells by confocal laser scanning microscopy (Figure 4A) as well as Western blot analysis using anti-BTK and anti-MBP antibodies (Figure 4B) confirmed the presence of the electroporated MBP-BTK protein. As shown in Figure 4C, introduction of wild-type BTK protein by electroporation rendered the BTK-deficient DT-40 cells resistant to the apoptotic effects of Fas-ligation, suggesting direct protein-protein interactions between BTK and members of the Fas signal transduction pathway as a possible mechanism for the anti-apoptotic function of BTK.

The downstream pro-apoptotic events initiated by the ligation of Fas or TNF receptor-1 are beginning to be illuminated (Fraser, A., Evan, G. (1996) *Cell* 85, 781-784; Kischkel, F.C., et al. (1995) *EMBO J.* 14, 5579-5588; Hu, S., Vincenz, et al.(1997) *J. Biol. Chem.* 272, 17255-17257; Deveraux, Q.L., et al. *(1997)Nature* 388, 300-304; Thome, M., et al. (1997)*Nature* 386, 517-521; Boldin, M.P., et al. (1995) *J. Biol. Chem.* 270, 7795-7798; Los, M., et al. (1995) *Nature* 375, 81-83; Boldin, M.P., et al. (1996) *Cell* 85, 803-815; and Chinnaiyan, A.M., et al. (1995) *Cell* 81, 505-512). Both Fas and TNF receptor-1 contain a homologous intracellular "death domain", which plays a pivotal role in ligand-dependent assembly of a pro-apoptotic death inducing signaling complex (DISC) (Kischkel, F.C., et al. (1995) *EMBO J.* 14,5579-5588). The death domains of p55 TNF receptor-1 and Fas/CD95 serve as docking sites that mediate ligand-dependent recruitment of and heteroassociation with other death domain containing multivalent adapter proteins: Fas associated protein with death domain (FADD) and receptor-interacting protein (RIP) in the case of CD95; and TNF receptor-1-associated death domain protein (TRADD) and RIP in the case of TNF receptor-1 (Fraser, A., Evan, G. (1996) *Cell* 85, 781-784; Kischkel, F.C., et al. (1995) *EMBO J.* 14, 5579-5588; and Chinnaiyan, A.M., et al. (1995) *Cell* 81, 505-512). FADD is the point of convergence between the Fas/CD95- and TNF receptor-1-linked apoptotic signal transduction pathways. Whereas Fas/CD95 directly recruits FADD, TNF receptor-1 binds TRADD, which then acts as an adapter protein to recruit FADD. The formation of CD95-FADD or TNF receptor-1-TRADD-FADD complexes following ligand binding are important for the induction of apoptosis. The assembly of a pro-apoptotic DISC is completed by the recruitment and concomitant activation of the cytosolic caspase FLICE, a member of the ICE protease family (Fraser, A., Evan, G. (1996) *Cell* 85, 781-784; Kischkel, F.C., et al. (1995) *EMBO J.* 14, 5579-5588; Hu, S., Vincenz, et al.(1997) *J. Biol. Chem. 272*, 17255-17257; Deveraux, Q.L., et al. (1997)*Nature* 388, 300-304; Thome, M., et al. (1997) *Nature* 386, 517-521; Boldin, M.P., et al. (1995) *J. Biol. Chem.* 270, 7795-7798; Los, M., et al. (1995) *Nature* 375, 81-83; Boldin, M.P., et al. (1996) *Cell* 85, 803-815; and Chinnaiyan, A.M., et al. (1995) *Cell* 81, 505-512). Recently, a number of proteins have been identified as inhibitors of Fas- as well as TNF receptor-1 induced apoptosis (Fraser, A., Evan, G. (1996) *Cell* 85, 781-784; Kischkel, F.C., et al. (1995) *EMBO J*. 14, 5579-5588; Hu, S., Vincenz, et al.(1997) *J. Biol. Chem.* 272, 17255-17257; Deveraux, Q.L., et al. (1997) *Nature* 388, 300-304; Thome, M., et al. (1997) *Nature* 386, 517-521). These proteins interact directly with FADD or FLICE, thereby interfering with DISC assembly and function. Notably, the death domain of Fas contains a conserved YXXL motif similar to the immunoreceptor tyrosine-based activation motif (ITAM) sequences as a potential binding site for SH2 containing proteins and Fas has recently been shown to associate with Fyn and Lck kinases as pro-apoptotic regulators which are required for induction of Fas-mediated apoptosis (Schlottmann, K.E., et al. *Leukocyte Biology* 60, 546-554; and Atkinson, E.A., et al. (1996) *J*. *Biol. Chem.* 271, 5968-5971). We therefore postulated that BTK could interact with Fas and prevent the assembly of a proapoptotic DISC after Fas-ligation.

We first investigated if BTK is capable of a physical association with Fas and other members of DISC by examining the Fas, FLICE, FADD, and TRADD immune complexes from the Nonidet P-40 lysates of untreated DT-40 cells for the presence of BTK. BTK was detected by Western blot analysis in Fas (but not the other) immune complexes by anti-BTK immunoblotting (Figure 5A). Similarly, Fas was detected by anti-Fas immunoblotting in BTK immune complexes from wild-type DT-40 cells as well as BTK-deficient DT-40 cells reconstituted with wild-type human *btk* gene (Figure 5B). The constitutive association of BTK with Fas protein was also found in the human B-cell precursor leukemia cell line NALM-6-UM1 (Figure 5C). Taken together, these results demonstrated that BTK is capable of associating with Fas protein and this physical association does not require prior engagement of the Fas receptor. As shown in Figure 5D, Fas is associated with FADD in BTK-deficient DT-40 cells, as evidenced by detection of Fas in FADD immune complexes and this physical interaction was markedly enhanced after Fas-ligation. In Fas-activated BTK-deficient DT-40 cells, Fas-associated FADD molecules could be detected by anti-FADD immunoblotting (Figure 5D). In contrast to BTK-deficient DT-40 cells, very little Fas-FADD association was found in untreated or anti-Fas treated BTK-deficient DT-40 cells reconstituted with wild-type human BTK (Figure 5B). Similarly, Fas-ligation failed to enhance the Fas-FADD association in human NALM-6-UM1 leukemia cells (Figure 5C). Thus, BTK associates with Fas and impairs its interaction with FADD, a protein that is essential for the recruitment and activation of FLICE by Fas during the apoptotic signal. While these results do not exclude the possibility that BTK may alter the fate of the apoptotic signal triggered by Fas ligation by multiple mechanisms including modulation of the function of positive or negative regulators of apoptotic signal transduction, they do provide at least one plausible explanation for the observed anti-apoptotic function of BTK.

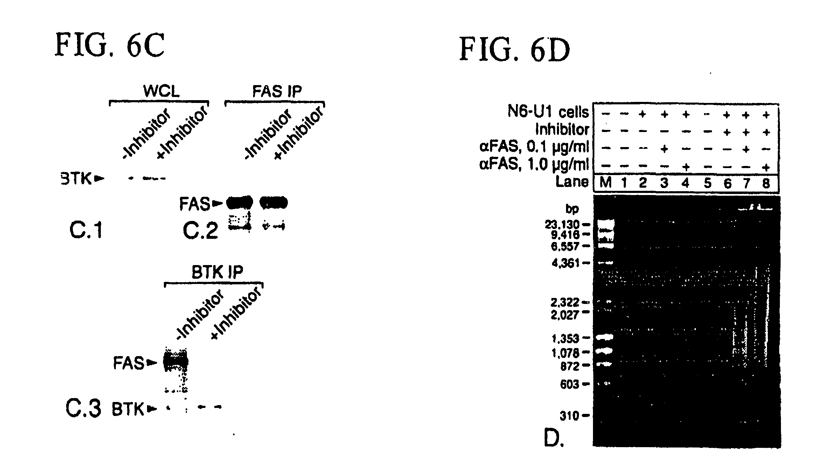

To further elucidate the physiologic significance of the observed BTK-Fas association in human leukemic B-cell precursors, we compared the sensitivities of BTK-positive NALM-6-UM1 human pre-B leukemia cell line and BTK-deficient RAMOS-1 human B-cell leukemia cell line to Fas-mediated apoptosis. As shown in Figure 6A, these two cell lines express similar levels of Fas protein. BTK-deficient RAMOS-1 cells underwent apoptosis after Fas ligation with the agonistic anti-Fas antibody but BTK-positive NALM-6-UM1 cells did not (Figure 6B). We next examined the effects of the leflunomide metabolite analog α-cyano-β-hydroxy-β-methyl -N-(2,5-dibromophenyl)propenamide (LMA-13), a potent inhibitor of BTK on BTK-Fas association and resistance to Fas-mediated apoptosis in NALM-6-UM1 cells. Anti-BTK and anti-Fas Western blot analyses of whole cell lysates from LMA-13-trested NALM-6-UM1 cells showed no reduction in BTK (Figure 6.C.1) or Fas protein (Figure 6.C.2) expression levels. There was substantially less Fas protein in the BTK immune complexes, providing direct evidence that inhibition of BTK by LMA-13 abrogates the BTK-Fas association (Figure 6C.3). Notably, a four hour treatment with LMA-13 did not induce apoptosis in NALM-6-UM1 cells but rendered these highly resistant human leukemia cells sensitive to Fas-mediated apoptosis (Figure 6D). These results provided further show that BTK is a physiologically important negative regulator of Fas-mediated apoptosis.

The ability of the BTK inhibitor LFM-A13 to abrogate the BTK-Fas association provided evidence that the kinase activity of BTK plays an important role for the formation of the BTK-Fas complexes. In order to further establish whether or not the association of BTK with Fas is dependent on its kinase activity, we next examined BTK-Fas interactions in BTK-deficient DT-40 cells reconstituted with either wild-type human BTK (BTK-, rBTK[WT]) or kinase inactive mutant (Arg⁵²⁵ to Gln) human BTK (BTK-, rBTK[K-]). As shown in Figure 7A, Western blot analysis of whole cell lysates from these two cell lines with anti-BTK or anti-Fas antibodies did not reveal any substantial differences (i.e., in both of two independent experiments, we observed slightly higher BTK and Fas expression levels in BTK-, rBTK[K-] cells ). Fas immune complexes from lysates of BTK-, rBTK[WT] cells contained BTK protein and this BTK-Fas association was further enhanced by treatment of cells with the anti-Fas antibody (Figure 7B, lanes 1 and 2). In contrast, no BTK protein was detectable in Fas immune complexes from BTK-, rBTK[K-] cells regardless of treatment with the anti-Fas antibody (Figure 7B, lanes 3 and 4). These results provide corroborating evidence that the association of BTK with Fas is dependent on the kinase activity of BTK.

We next performed binding experiments with full length MBP-BTK and truncated MBP-BTK and GST-BTK fusion proteins corresponding to various domains of BTK (Figure 8A-C) to elucidate the structural requirements for BTK association with Fas. MBP-BTK 1-659 (full length BTK) as well as MBP-BTK 408-659 ("BTK kinase domain") and MBP-BTK 2-137 ("BTK PH domain") were able to bind and pull down Fas from lysates of BTK-deficient DT-40 cells (Figure 8D), human NALM-6 pre-B leukemia cells (Figure 8E), and KL2 human EBV-transformed B-lymphoblastoid cells (Figure 8F). However, Fas did not bind to the control MBP-BTK 519-567 fusion protein corresponding to a truncated kinase domain containing the Y551 transphosphorylation site (C5 in Figure 7D), GST-BTK 219-377 containing the SH3 plus SH2 domains, GST-BTK 219-268 corresponding to the SH3 domain, or GST-BTK 281-377 corresponding to SH2 domain (the latter two were used only with lysates from BTK-deficient DT-40 cells) (Figure 8D-F).

Although the crystal structure of full length BTK has not been reported, the recently published structures of the PH domain and BTK motif (Hyvonen, M., Saraste, M. (1997) *EMBO J.* 16, 3396-3404) provide useful information applicable to the binding capability of BTK and its PH domain. FADD has been reported to interact with the cytoplasmic domain of Fas, which is largely composed of a death domain consisting of six antiparallel α-helices assembled from residues 230-314 (Huang, B., et al. (1996) *Nature* 384, 638-641). The YXXL sequence of the Fas death domain has been speculated to resemble ITAMs and be recognized by an SH2 domain of a PTK upon tyrosine phosphorylation or by other mechanisms (Schlottmann, K.E., et al. *Leukocyte Biology* 60, 546-554; and Atkinson, E.A., et al. (1996) *J. Biol. Chem.* 271, 5968-5971). An analysis of the conformation of this YXXL sequence shows that it is located in the middle of an α-helix and unless a substantial conformational change of that α-helix would occur to make the tyrosine residue more accessible, it may be too rigid for interaction with a PTK. Thus, the structural geometry of the YXXL sequence would likely prevent Fas and BTK to adopt a binding mode such as that of CD3-ε ITAM/ZAP-70 as was suggested by Atkinson, E.A., et al. (1996) *J. Biol. Chem.* 271, 5968-5971. The inability of the BTK SH2 domain to pull down Fas from whole cell lysates further supports this notion. How then does BTK associate with Fas? BTK and Fas may associate via complementary electrostatic attractions and hydrogen bonding interactions that could involve the previously reported charged residues on the surfaces of the α-helices of the Fas death domain. This association could be mediated by a third protein that forms an interface between Fas and BTK. The importance of the SH2 and kinase domains of BTK for its anti-apoptotic function prompts the hypothesis that a tyrosine phosphorylated substrate of BTK may provide such an interface.

The ability of BTK to inhibit the pro-apoptotic effects of Fas-ligation prompts the hypothesis that apoptosis of developing B-cell precursors during normal human B-cell ontogeny may be reciprocally regulated by Fas and BTK. The absence of BTK or mutations in its kinase, PH, and SH2 domains could lead to inappropriate apoptotic cell death of pre-B cells leading to the phenotype of XLA (Rawlings, D.J., Witte, O.N. (1994) *Immunol. Rev.* 138, 105-119; Tsukada, S., et al. (1993) *Cell* 72, 279-290; and Vetrie, D. (1993) *Nature* 361, 226-233). Inappropriate apoptosis may underlie the pathogenesis as well as drug resistance of human leukemias and lymphomas, which makes control of apoptosis an important potential target for therapeutic intervention. The fate of leukemia/lymphoma cells exposed to chemotherapeutic agents (e.g. vincristine, daunorubicin, or taxol) may reside in the balance between the opposing proapoptotic effects of caspases activated by DISC and an upstream negative regulatory mechanism involving BTK and/or its substrates. Therefore, inhibitors of BTK are likely to enhance the drug sensitivity of B-lineage leukemia/lymphoma cells.

### Example 2: Synthesis of specific leflunomide metabolite analogs.

**Chemistry.** All chemicals were purchased from Aldrich (Milwaukee, WI) and were used without further purification. Except where noted, each reaction vessel was secured with a rubber septum, and the reaction was performed under nitrogen atmosphere. ¹H and ¹³C NMR spectra were obtained on a Varian Mercury 300 instrument spectrometer (Palo Alto, CA) at ambient temperature in the solvent specified. Melting points were determined using a Fisher-Johns melting point apparatus and are uncorrected. FT-IR spectra were recorded on a Nicolet Protege 460 spectrometer (Madison, WI). GC/MS spectra were obtained on a HP 6890 GC System (Palo Alto, CA) equipped with a HP 5973 Mass Selective Detector.

MS (EI) spectra were obtained on an HP Series 1100 LL/MSD.

The general synthetic scheme for the preparations of LFM, and LFM-A1 - LFM-AL14 is illustrated in Figure 19 with the inclusion of LFM, LFM-A1 to LFM-A12 and LFM-A14 for comparative purposes only. Cyanoacetic acid 1 was coupled with the desired aniline 2 in the presence of diisopropylcarbodiimide (DIC) to form 3. Compound 3 was treated with NaH and then acylated with acetyl chloride to afford LFM or LFM-A1 to LFM-A 14.

### General Synthetic Procedure

1,3-Diisopropylcarbodiimide (1.75 g; 13.9 mmol) was added to a solution of cyanoacetic acid 1 (1.70 g; 20.0 mmol) and the desired substituted-aniline 2 (12.6 mmol) in tetrahydrofuran (25 mL) at 0°C. The mixture was stirred for 12 hours at room temperature. The resulting urea precipitate (reaction side product) was removed by filtration and the filtrate was partitioned between ethyl acetate and 0.5 N HCl. The organic layer was sequentially washed with brine twice, dried over anhydrous Na₂SO₄ and concentrated by rotary-evaporation. The crude solid product was recrystallized from ethyl alcohol to give pure 3. See Kuo, E. A., et al. (1996) *J.* *Med. Chem.* 39(23), 4608-21; and Sjogren, E. R., et al. (1991) *J. Med. Chem. 34,* 3295-3301.

Sodium hydride (0.93 g; 60% in mineral oil; 23.2 mmol) was added slowly to the solution of 3 (12.0 mmol) in tetrahydrofuran (40 mL) at 0°C. After stirring for 30 minutes at 0°C, acetyl chloride (1.04g; 13.2 mmol) was added to the reaction mixture. The reaction was continued for another hour at room temperature and was quenched by the addition of acetic acid (2 mL). The mixture was poured into ice water (100 mL) containing 2.5 mL of hydrochloric acid to precipitate the crude product, which was collected by filtration and washed with water. The final pure product was obtained by recrystallization.

### Physical data.

In the following sections, physical data of all compounds other than LFM-A13, (i.e. LFM, LFM-A1 to LFM-A12, and LFM-A14) are listed for comparative purposes only:
**α-Cyano- β-hydroxy-β-methyl-*N*-[4-(trifluoromethyl)phenyl]-propenamide (LFM).** mp: 230 - 233°C; IR (KBr): 3303, 2218, 1600 and 1555 cm⁻¹; ¹H NMR (DMSO-*d*₆): δ 11.01 (s, 1H, NH), 7.75 (d, *J*= 8.4 Hz, 2H, ArH), 7.64 (d,*J*= 8.4 Hz, 2H, ArH), 2.22 (s, 3H, CH₃); GC/MS *m*/*z* 270 (M⁺), 161, 142, 111.
**α-Cyano-β-hydroxy-β-methyl-*N*-(4-bromophenyl)propenamide (LFM-A1).** mp: 213 - 214°C; IR (KBr): 3288, 2228, 1615, 1555 cm⁻¹; ¹H NMR (DMSO-*d*₆): δ 10.51 (s, 1H, NH), 7.49 (s, 4H, ArH), 2.25 (s, 3H, CH₃); MS (El) *m*/*z* 280 (M⁺).
**α-Cyano-β-hydroxy-β-methyl-*N*-(4-chlorophenyl)propenamide (LFM-A2).** mp: 209 - 211°C; IR (KBr): 3298, 2223, 1598 and 1552 cm⁻¹; ¹H NMR (DMSO-*d*₆): δ 10.48 (s, 1H, NH), 7.54 (d, *J* = 8.7 Hz, 2H, ArH), 7.45 (s br, 1H, OH), 7.36 (d, *J* = 8.7 Hz, 2H, ArH), 2.25 (s, 3H, CH₃); MS (CI) *m*/*z* 236 (M⁺), 129, 127.
**α-Cyano-β-hydroxy-β-methyl-*N*-(4-fluorophenyl)propenamide (LFM-A3). mp:** 165 - 166°C; IR (KBr): 3298, 2218, 1610 and 1560 cm⁻¹; ¹H NMR (DMSO-*d*₆): δ 10.33 (s, 1H, NH), 7.80 (s br, 1H, OH), 7.53 (m, 2H, ArH), 7.16 (m, 2H, ArH), 2.26 (s, 3H, CH₃); GC/MS *m*/*z* 220 (M⁺), 111.
**α-Cyano-β-hydroxy-β-methyl-*N*-[2-(trifluoromethyl)phenyl]-propenamide (LFM-A4).** mp: 61 - 63°C; IR (KBr): 3435, 2209, 1619, 1952 and 1548 cm⁻¹; ¹H NMR (DMSO-*d*₆): δ 10.99 (s, 1H, NH), 8.03 (d, *J* = 7.5 Hz, 1H, ArH), 7.67 (d, *J* = 7.5 Hz, 1H, ArH), 7.60 (dd, *J =* 7.5, 7.5 Hz, 1H, ArH), 7.29 (dd, *J=* 7.5, 7.5 Hz, 1H, ArH) 5.71 (s br, 1H, OH), 2.20 (s, 3H, CH₃); GC/MS *ms*/*z* 270 (M⁺), 161, 141, 114.
**α-Cyano-β-hydroxy-β-methyl-*N*-(2-bromophenyl)propenamide (LFM-A5).** mp: 98 - 100°C; IR (KBr): 3351,2214, 1609, 1585 and 1536 cm⁻¹; ¹H NMR (DMSO-*d*₆): δ 10.76 (s, 1H, NH), 8.06 (dd, *J=* 8.1, 1.5 Hz, 1H, ArH), 7.62 (dd, *J* = 8.1, 1.5 Hz, 1H, ArH), 7.33 (m, 1H, ArH), 7.03 (m, 1H, ArH), 6.60 (s br, 1H, OH), 2.22 (s, 3H, CH₃); ); MS (EI) *m*/*z* 280 (M⁺), 173, 171.
**α-Cyano-β-hydroxy-β-methyl-*N*-(2-chlorophenyl)propenamide (LFM-A6).** mp: 93 - 94°C; IR (KBr): 3372, 2208, 1644, 1621 and 1587 cm⁻¹; ¹H NMR (DMSO-*d*₆): δ 10.96 (s, 1H, NH), 8.16 (d, *J* = 8.1 Hz, 1H, ArH), 7.46 (dd, *J* = 7.5, 1.5 Hz, 1H, ArH), 7.29 (m, 1H, ArH), 7.08 (m, 1H, ArH), 2.22 (s, 3H; CH₃); MS (CI) *m*/*z* 236 (M⁺), 129, 127.
**α-Cyano-β-hydroxy-β-methyl-*N*-(2-fluorophenyl)propenamide (LFM-A7).** mp: 118 -119°C; IR (KBr): 3409, 2212, 1613, 1591 and 1532 cm⁻¹; ¹H NMR (DMSO-*d*₆): Λ 10.70 (s, 1H, NH), 7.91 (m, 1H, ArH), 7.23 (m, 1H, ArH), 7.13 (m, 2H, ArH), 7.10 (s br, 1H, OH), 2.22 (s, 3H, CH₃); GC/MS *m*/*z* 220 (M⁺), 111.
**α-Cyano-β-hydroxy-β-methyl-*N*-[3-(trifluoromethyl)phenyl]-propenamide (LFM-A8).** mp: 182 - 184°C; IR (KBr): 3303, 2221, 1619 and 1572 cm⁻¹; ¹H NMR (DMSO-*d*₆): δ 10.79 (s, 1H, NH), 8.05 (s br, 1H, OH) 8.04 (s, 1H, ArH), 7.75 (d, *J* = 8.1 Hz, 1H, ArH), 7.53 (dd, *J*= 8.1, 7.5 Hz, 1H, ArH), 7.42 (d, *J* = 7.5 Hz, 1H, ArH), 2.24 (s, 3H, CH₃); GC/MS *m*/*z* 270 (M⁺), 161.
**α-Cyano-β-hydroxy-β-methyl-*N*-(3-bromophenyl)propenamide (LFM-A9).** mp: 184 - 185°C; IR (KBr): 3303, 2228, 1610, 1595 and 1550 cm⁻¹; ¹H NMR (DMSO-*d*₆): δ 10.56 (s, 1H, NH), 7.89 (m, 1H, ArH), 7.47 (m, 1H, ArH), 7.28 (m, 2H, ArH), 6.37 (s br, 1H, OH), 2.26 (s, 3H, CH₃); MS (EI) *m*/*z* 282 (M⁺ + H, ⁸¹Br), 280 (M⁺ + H, ⁷⁹Br), 173, 171.
**α-Cyano-β-hydroxy-β-methyl-*N*-(3-chlorophenyl)propenamide (LFM-A10).** mp: 184 - 187°C; IR (KBr): 3293, 2221, 1610, 1595 and 1557 cm⁻¹; ⁻H NMR (DMSO-*d*₆): Λ 10.61 (s, 1H, NH), 7.76 (m, 1H, ArH), 7.42 (m, 1H, ArH), 7.33 (dd, J = 8.1, 8.1 Hz, 1H, ArH), 7.16 (m, 1H, ArH), 6.84 (s br, I H, OH), 2.25 (s, 3H, CH₃); MS (CI) *m*/*z* 239 (M⁺ + H, ³⁷Cl), 237 (M⁺ + H³⁵Cl), 129,127.
**α-Cyano-β-hydroxy-β-methyl-*N*-(3-fluorophenyl)propenamide (LFM-A11).** mp: 136 - 138°C; IR (KBr): 3297, 2221, 1613, 1597 and 1567 cm⁻¹; ¹H NMR (DMSO-*d*₆): δ 10.54 (s, 1H, NH), 7.54 (m, 1H, ArH), 7.33 (m, 2H, ArH), 6.93 (m, 1H, ArH), 2.27 (s, 3H, CH₃); GC/MS *m*/*z* 220 (M⁺), 111.
**α-Cyano-β-hydroxy-β-methyl-*N*-[4-(trifluoromethoxy)phenyl]-propenamide (LFM-A12).** mp: 182 - 183°C; IR (KBr): 3308, 2213, 1625 and 1580 cm⁻¹; ¹H NMR (DMSO-*d*₆): δ 10.57 (s, 1H, NH), 7.90 (s br, 1H, OH), 7.64 (d, *J* = 8.7 Hz, 2H, ArH), 7.32 (d, *J*= 8.7 Hz, 2H, ArH), 2.25 (s, 3H, CH₃); GC/MS *m*/*z* 286 (M⁺), 177, 108.
**α-Cyano-β-hydroxy-β-methyl-*N*-(2,5-dibromophenyl)-propenamide (LFM-A13)**. mp: 148 - 150°C; IR (KBr): 3353, 2211, 1648 and 1590 cm⁻¹; ¹H NMR (DMSO-*d*₆): δ 11.41 (s, 1H, NH), 8.57 (d,*J*= 2.4 Hz, 1H, ArH), 7.55 (d,*J*= 8.7 Hz, 1H, ArH), 7.14 (dd, *J* = 8.7, 2.4 Hz, 1H, ArH), 7.10 (s br, 1H, OH), 2.17 (s, 3H, CH₃); MS (EI) *m*/*z* 362 (M⁺ + 4), 360 (M⁺ + 2), 358 (M⁺), 253, 251, 249, 150.
**α-Cyano-β-hydroxy-β-methyl-*N*-(phenyl)propenamide (LFM-A14).** mp: 134 - 135°C; IR (KBr): 3281, 2214, 1605, 1579 and 1554 cm⁻¹; ¹H NMR (DMSO-*d*₆): δ 10.33 (s, 1H, NH), 7.51 (d, *J* = 7.5 Hz, 2H, ArH), 7.40 (s br, 1H, OH), 7.31 (dd, *J* = 7.5, 7.5 Hz, 2H, ArH), 7.11 (m, 1H, ArH), 2.26 (s, 3H, CH₃); GM/MS *m*/*z* 202 (M⁺), 93.

Using procedures similar to the general procedure identified above, the following compounds of formula I were also prepared.
**α-Cyano-β-hydroxy-β-methyl-*N*-[4-(methylsulfonyl)phenyl]-propenamide;** prepared by oxidation of the corresponding 4-methylthio compound with peracetic acid in acetic acid; mp: 205-206°C; ¹H NMR (DMSO-*d*₆): δ 11.26 (s, 1), 7.81 (d, 2), 7.76 (d, 2), 3.15 (s, 3), 2.19 (s, 3); IR (KBr): 3309, 2225, 1643 and 1586 cm⁻¹; MS (EI) *m*/*z* 281.0 (M + H⁺),172.1.
**α-Cyano-β-hydroxy-β-methyl-*N*-[3-(methylsulfonyl)phenyl]-propenamide;** prepared by oxidation of the corresponding 3-methylthio compound with peracetic acid in acetic acid; mp: 213-214°C; ¹H NMR (DMSO-*d*₆): δ 10.98 (s, 1), 8.18 (m, 1), 7.82 (m, 1), 7.60 (m, 2), 3.18 (s, 3), 2.23 (s, 3); IR (KBr): 3278, 2231, 1607 and 1555 cm⁻¹; MS (EI) *m*/*z* 281.0 (M + H⁺), 172.1.
**α-Cyano-β-hydroxy-β-methyl-*N*-[3-bromo-4-(trifluoromethoxy)phenyl]propenamide;** mp: 178-179°C; ¹H NMR (DMSO-*d*₆): δ 11.03 (s, 1), 8.12 (d, 1), 7.55 (dd, 1) , 7.45 (m, 1), 5.53 (s, 1), 2.20 (s, 3); IR (KBr): 3304, 2350, 1620 and 1602 cm⁻¹; MS (EI) *m*/*z* 365.0 (M + H⁺),255.9.
**α-Cyano-β-hydroxy-β-methyl-*N*-(2,4-dibromophenyl)-propenamide;** mp: 181-181°C; ¹H NMR (DMSO-*d*₆): δ 11.03 (s, 1), 8.14 (m, 1), 7.84 (d, 1),7.51 (dd, 1), 5.65 (s, 1), 2.20 (s, 3); IR (KBr): 3360, 2205, 1591 and 1530 cm⁻¹;MS (EI) *m*/*z* 361.0 (M + H⁺),249.9.
**α-Cyano-β-hydroxy-β-methyl-*N*-(2,4-dichlorophenyl)-propenamide;** mp: 143-144°C; ¹H NMR (DMSO-*d*₆): δ 11.23 (s, 1), 8.29 (m, 1), 7.60 (d, 1) , 7.35 (dd, 1), 5.17 (s, 1), 2.18 (s, 3); IR (KBr): 3371, 2210, 1601 and 1540 cm⁻¹;MS (EI) *m*/z 271.0 (M + H⁺), 162.0.
**α-Cyano-β-hydroxy-β-methyl-*N*-(2,5-dichlorophenyl)-propenamide;** mp: 143-144°C; ¹H NMR (DMSO-*d*₆): δ 11.70 (s, 1), 8.51 (d, 1), 7.45 (d, 1) , 7.05 (dd, 1), 4.97 (s, 1), 2.14 (s, 3); IR (KBr): 3370, 2215, 1581 and 1528 cm⁻¹;MS (EI) *m*/*z* 271.0 (M + H⁺), 162.0.
**α-Cyano-β-hydroxy-β-methyl-*N*-(3,4-dichlorophenyl)-propenamide;** mp: 216-217°C; ¹H NMR (DMSO-*d*₆): δ 10.74 (s, 1), 7.95 (m, 1), 7.54 (m, 1), 7.47 (m, 1), 5.64 (s, 1), 2.23 (s, 3); IR (KBr): 3319, 2225 and 1612 cm⁻¹;MS (EI) *m*/*z* 271.0 (M + H⁺), 162.0.

Using a BTK inhibition assay similar to the one described in Table 1, the above compounds were found to act as BTK inhibitors, generally having IC₅₀'s of 20 µm/mL or less. The compounds were also found to sensitize cells to apoptosis with vincristine and ceramide using a procedure similar to that described in Example 3.

**Example 3:** The following example provides information of how specific inhibitors of BTK can be designed. A homology model for the kinase domain of BTK is disclosed, which is useful for the design and confirmation of inhibitors of BTK. The use of the homology model to identify specific compounds, including a novel leflunomide metabolite analog that is a potent and selective inhibitor of BTK is also disclosed.

In an effort to design potent inhibitors of the anti-apoptotic tyrosine kinase BTK as antileukemic agents with apoptosis promoting and chemosensitizing properties, a three-dimensional homology model of the BTK kinase domain was constructed, as described more completely below. Modeling studies revealed a distinct rectangular binding pocket near the hinge region of the BTK kinase domain with Leu⁴⁶⁰, Tyr⁴⁷⁶, Arg⁵²⁵ and Asp⁵³⁹ residues occupying the comers of the rectangle. The dimensions of this rectangle are approximately 18Å x 8Å x 9Å x 17Å and the thickness of the pocket is approximately 7Å.

Advanced docking procedures were employed for the rational design of leflunomide metabolite (LFM) analogs with a high likelihood to bind favorably to the catalytic site within the kinase domain of BTK. The compound LFM-A13, for which we calculated a Kᵢ value of 1.4 µM, inhibited human BTK *in vitro* with an IC₅₀ value of 17.2 ± 0.8 µM. Similarly, LFM-A13 inhibited recombinant BTK expressed in a baculovirus expression vector system with an IC₅₀ value of 2.5 µM. The energetically favorable position of LFM-A 13 in the binding pocket is such that its aromatic ring is close to Tyr⁴⁷⁶, and its substitutent group is sandwiched between residues Arg⁵²⁵ and Asp⁵³⁹. In addition, LFM-A13 is capable of favorable hydrogen bonding interactions with BTK via Asp⁵³⁹ and Arg⁵²⁵ residues.

Besides its remarkable potency in BTK kinase assays, LFM-A13 was also discovered to be a highly specific inhibitor of BTK. Even at concentrations as high as 100 µg/ml (~278 µM), this novel inhibitor did not affect the enzymatic activity of other protein tyrosine kinases, including JAK1, JAK3, HCK, EGF-Receptor Kinase (EGFR), and Insulin-Receptor Kinase (IRK).

In accordance with the anti-apoptotic function of BTK, treatment of BTK⁺ B-lineage leukemic cells with LFM-A13 enhanced their sensitivity to ceramide-or vincristine-induced apoptosis.

### Crystal Structures of Leflunomide Metabolite and Its Analogs

The leflunomide metabolite (LFM) and two of its analogs (LFM-A12, LFM-A13) were crystallized using various solvents by evaporation or liquid-liquid diffusion. LFM and LFM-A12 were crystallized and subsequently analysed for comparative purposes only. X-ray data from single crystals were collected using a SMART CCD area detector (Bruker Analytical X-ray Systems, Madison, WI) with MoKI radiation (Σ = 0.7107 Å). The space group was determined based on systematic absences and intensity statistics. A direct methods solution provided most of the non-hydrogen atoms from the electron density map. Several full-matrix least squares/difference Fourier cycles were performed to locate the remaining non-hydrogen atoms. All non-hydrogen atoms were refined with anisotropic thermal parameters. Hydrogen atoms were placed in ideal positions and refined as riding atoms with relative isotropic temperature factors. The structure was refined using full-matrix least-squares on F². Crystal structure calculations were performed using a Silicon Graphics INDY R4400-SC computer (Silicon Graphics Inc., Mountain View, CA) or a Pentium computer using the SHELXTL V 5.0 suite of programs (Sheldrick, G., 5.0 Ed., Bruker Analytical X-ray Systems, Madison, WI).

### Construction of the Homology Model for the Kinase Domain of BTK.

A homology model of BTK was constructed using crystal structures of homologous kinase domains of protein kinases HCK, FGFR, IRK, and cAPK (Sicheri, F., Moarefi, I., and Kuriyan, J. (1997) *Nature* 385(6617), 602-9; Mohammadi, M., et al. (1997) *Science* 276(5314), 955-60; Hubbard, S. R. (1997) *The E. M. B. O. Journal* 16(18), 5572-5581; and Zheng, J., et al. (1993) *Acta Cryst.* D49, 362-365). The homology modeling of BTK was carried out by first obtaining the protein sequence ofBTK (Swiss-Prot # Q06187, Univ. of Geneva, Geneva, Switzerland) from GenBank (National Center for Biotechnology Information, Bethesda, MD). Next, the most reasonable sequence alignment between the BTK kinase and a coordinate template was determined. This was done by first superimposing the CI coordinates of the kinase domains of HCK, FGFR, IRK, and cAPK using the InsightII program ((1996), Molecular Simulations, Inc., San Diego, CA) to provide the best overall structural comparison. All four sequences were then aligned based on the superimposition of their structures (amino acid sequences were aligned together if their CI positions were spatially related to each other). The sequence alignment accommodated such features as loops in a protein that differed from the other protein sequences. The structural superimposition was done using the Homology module of the InsightII ((1996), Molecular Simulations, Inc., San Diego, CA) program and a Silicon Graphics INDIGO2 computer (Silicon Graphics Inc., Mountain View, CA). The sequence alignment was manually adjusted based on the previously mentioned considerations, and produced a sequence variation profile for each superimposed CI position. The sequence variation profile served as a basis for the next procedure, which was sequence alignment of all four proteins with BTK kinase. In this procedure, the sequence of BTK kinase was read into the program and manually aligned with the four known kinase proteins based on the sequence variation profile described previously. Next a set of 3D coordinates was assigned to the BTK kinase sequence using the 3D coordinates of HCK as a template, which employed the Homology module within the InsightII program ((1996), Molecular Simulations, Inc., San Diego, CA). The coordinates for a loop region where a sequence insertion occurs (relative to HCK without the loop) was chosen from a limited number of possibilities automatically generated by the program and manually adjusted to a more ideal geometry using the program CHAIN (Sack, J. S. (1988) *J. Mol. Graphics* 6, 244-245). Finally, the constructed model of BTK was subjected to energy minimization using the X-plor program (Brunger, A. T. (1992), New Haven, CT) so that any steric strain introduced during the model-building process could be relieved. The model was screened for unfavorable steric contacts and if necessary such side chains were remodeled either by using a rotamer library database or by manually rotating the respective side chains. The final homology model of the BTK kinase domain had an RMS deviation of 0.01 Å from ideal bond lengths and 2.2° from ideal bond angles after energy minimization. The homology model of BTK was then used, in conjunction with model coordinates of LFM and its analogs (which were later compared with crystal structures), for modeling studies of the BTK/inhibitor complexes.

### Docking Procedure using Homology Model of BTK Kinase Domain.

Modeling of the BTK/LFM analog complexes was done using the Docking module within the program INSIGHTII and using the Affinity suite of programs for automatically docking a ligand to the receptor. BTK in complex with LFM or LFM-A12 was modelled for comparative purposes only. Energy-minimized coordinates for each LFM molecule were generated and interactively docked into the ATP binding site of BTK based on the position of quercetin in the HCK/quercetin crystal structure (Sicheri, F., et al., J. (1997) *Nature* 385 (6617), 602-9). The hydrogen atoms on the kinase domain of BTK were generated and potentials were assigned to both receptor and ligand prior to the start of the docking procedure. The docking method in the InsightII program used the CVFF force field and a Monte Carlo search strategy to search for and evaluate docked structures. While the coordinates for the bulk of the receptor were kept fixed, a defined region of the binding site was allowed to relax, thereby allowing the protein to adjust to the binding of different inhibitors. A binding set was defined within a distance of 5Å from the inhibitor, allowing residues within this distance to shift and/or rotate to energetically favorable positions to accommodate the ligand. An assembly was defined consisting of the receptor and inhibitor molecule and docking was performed using the fixed docking mode. Calculations approximating hydrophobic and hydrophilic interactions were used to determine the ten best docking positions of each LFM analog in the BTK catalytic site. The various docked positions of each LFM analog was qualitatively evaluated using Ludi (Bohm, H. J. (1992) *J. Comput. Aided Mol. Des.* **6**(6), 593-606; and Bohm, H. J. (1994) *J. Comput. Aided Mol. Des.* 8(3), 243-56) in INSIGHTII which was used to estimate a binding constant (Kᵢ) for each compound in order to rank their relative binding capabilities and predicted inhibition of BTK. The Kᵢ trends for the LFM analogs were compared with the trend of the experimentally determined tyrosine kinase inhibition IC₅₀ values for the compounds, in order to elucidate the structure-activity relationships (SAR) determining the potency of LFM analogs.

### Recombinant Baculovirus Construction and Protein Expression.

Sf21 (IPLB-SF21-AE) cells (Vassilev, A., et al. (1998) *J. Biol. Chem.,* 274, 1646-1656) derived from the ovarian tissue of the fall armyworm *Spodotera frugiperda,* were obtained from Invitrogen and maintained at 26-28°C in Grace's insect cell medium supplemented with 10% FBS and 1.0% antibiotic/antimycotic (GIBCO-BRL). Stock cells were maintained in suspension at 0.2 - 1.6 x 10⁶/ml in 600 ml total culture volume in 1 L Bellco spinner flasks at 60-90 rpm. Cell viability was maintained at 95-100% as determined by trypan blue dye exclusion.

Recombinant baculovirus containing the murine *BTK* gene was constructed as described (Vassilev, A., et al. (1998) *J. Biol. Chem.,* 274, 1646-1656). In brief, the gene encoding BTK was excised from pBluescript SKII⁺ vector (Stratagene, La Jolla, Ca.) digestion with BamHI and this fragment was then ligated into pFastBac1 (Gibco-BRL). The resulting vector, pFastBac1-BTK, was then used to generate the recombinant baculovirus by site-specific transposition in *E. coli* DH10Bac cells (Gibco-BRL) that harbor a baculovirus shuttle vector (bacmid), bMON 14272. The resulting recombinant bacmid DNA was introduced into insect cells by transfection with the standard liposome-mediated method using Cellfectin reagent (Gibco-BRL). Four days later, transfection supernatants were harvested for subsequent plaque purification and analyzed as above. Kinase-dead BTK was generated as described (Vassilev, A., et al. (1998) *J. Biol. Chem.,* 274, 1646-1656) and cloned into the baculovirus expression vector as described above for wild type BTK. Baculovirus expression vectors for JAK1 and JAK3 kinases were constructed and introduced into insect cells, as previously reported (Goodman, P. A., et al. (1998) *J. Biol. Chem.* 273, 17742-48).

### Immunoprecipitation of Recombinant Proteins from Insect Cells.

Sf21 cells were infected with a baculovirus expression vector for BTK, JAK 1, or JAK3, as indicated in brief description of the figures. Cells were harvested, lysed (10mM Tris pH7.6, 100mM NaCl, 1% Nonidet P-40, 10% glycerol, 50mM NaF, 100mM Na₃VO₄, 50µg/ml phenylmethylsulfonyl fluoride, 10 µg/ml aprotonin, µg/ml leupeptin), and the kinases were immunoprecipitated from the lysates, as reported (Vassilev, A., et al. (1999) *J. Biol. Chem.,* 274, 1646-1656). Antibodies used for immunoprecipitations from insect cells are as follows: Polyclonal rabbit anti-BTK serum, (Mahajan, S., et al. (1995) *Mol. Cell. Biol.* 15, 5304-11) polyclonal rabbit anti-JAK1 (HR-785), cat# sc-277, rabbit polyclonal IgG affinity purified, 0.1 mg/ml, Santa Cruz Biotechnology, and polyclonal rabbit anti-JAK3 (C-21, cat # sc-513, rabbit polyclonal IgG affinity purified, 0.2 mg/ml, Santa Cruz Biotechnology). Kinase assays were performed following a 1 hour exposure of the immunoprecipitated tyrosine kinases to the test compounds, as described in detail elsewhere (Mahajan, S., et al. (1995) *Mol. Cell. Biol.* 15, 5304-11; and Uckun, F. M., et al. (1996) *Science* 22, 1096-1100). The immunoprecipitates were subjected to Western blot analysis as previously described (Vassilev, A., et al. (1998) *J. Biol. Chem.,* 274, 1646-1656).

### Cell lines, Reagents, and Biochemical Assays.

The establishment and characterization of DT40 lymphoma B cell line as well as BTK-deficient DT40 and its derivatives reconstituted with wild-type or mutant human BTK have been previously reported (Uckun, F. M., et al. (1996) *Science* 22, 1096-1100). Equal amounts of BTK protein were detected by Western blot analysis in all of the BTK-deficient DT-40 clones transfected with wild-type or mutated human *BTK* genes but no BTK protein was detectable in the untransfected BTK-deficient DT-40 cells (Uckun, F. M., et al. (1996) *Science* 22, 1096-1100). All cell lines derived from the chicken B-cell line DT40 were maintained in RPMI 1640 medium supplemented with 10% heat-inactivated fetal bovine serum, 1% heat-inactivated chicken serum, 2 mM glutamine, penicillin, and strepotmycin. Cells were grown at 37°C in a 5% CO₂ water saturated atmosphere. The BTK positive human B-lineage leukemia cell lines NALM-6 and ALL-1 were maintained in RPMI 1640 medium supplemented with 10% heat-inactivated fetal bovine serum (Uckun, F. M., et al. (1995) *Science* **267**, 886-91). COS-7 simian kidney cell line and HepG2 human hepatoma cell line were obtained from ATCC.

Antibodies directed against BTK, JAK1, JAK3, and HCK have been described previously (Mahajan, S., et al. (1995) *Mol. Cell. Biol.* 15, 5304-11; Vassilev, A., et al. (1998) *J. Biol. Chem.,* 274, 1646-1656; Goodman, P. A., et al. (1998) *J*. *Biol. Chem.* 273, 17742-48; and Uckun, F. M., et al. (1996) *Science* 22, 1096-1100). Polyclonal antibodies to BTK were generated by immunization of rabbits with glutathione S-transferase (GST) fusion proteins (Pharmacia Biotech Inc.) containing the first 150 amino acids of BTK. The monoclonal anti-Fas antibody (F22120) was obtained from the Transduction Laboratories, Inc. (Lexington, KY). Immunoprecipitations, immune-complex protein kinase assays, and immunoblotting using the ECL chemiluminescence detection system (Amersham Life Sciences) were conducted as described previously (Mahajan, S., et al. (1995) *Mol. Cell. Biol.* 15, 5304-11; Vassilev, A., et al. (1998) *J. Biol. Chem.,* 274, 1646-1656; Goodman, P. A., et al. (1998) *J*. *Biol. Chem.* 273, 17742-48; and Uckun, F. M., et al. (1996) *Science* 22, 1096-1100). Following electrophoresis, kinase gels were dried onto Whatman 3M filter paper and subjected to phosphoimaging on a Molecular Imager (Bio-Rad, Hercules, CA) as well as autoradiography on film. Similarly, all chemiluminescent BTK Western blots were subjected to three dimensional densitometric scanning using the Molecular Imager and Imaging Densitometer using the Molecular Analyst/Macintosh version 2.1 software following the specifications of the manufacturer (Bio-Rad). For each drug concentration, a BTK kinase activity index was determined by comparing the ratios of the kinase activity in phosphorimager units (PIU) and density of the protein bands in densitometric scanning units (DSU) to those of the baseline sample and using the formula: Activity Index = [PIU of kinase band/DSU of BTK protein band]_{test sample}: [PIU of kinase band/DSU of BTK protein band]_{baseline control sample}. GST-IGα was sometimes used as an exogenous substrate for BTK immune-complex protein kinase assays, as described (Mahajan, S., et al. (1995) *Mol. Cell. Biol.* 15, 5304-11). Horse radish peroxidase-conjugated sheep anti-mouse, donkey anti-rabbit secondary antibodies and ECL reagents were purchased from Amersham (Oakbrook, IL). For insulin receptor kinase (IRK) assays, HepG2 human hepatoma cells grown to approximately 80% confluency were washed once with serum-free DMEM and starved for 3 hour at 37° in a CO₂ incubator. Subsequently, cells were stimulated with insulin (Eli Lilly,cat# CP-410;10 units/ ml/10x10⁶ cells) for 10 minutes at room temperature. Following this IRK activation step, cells were washed once with serum free medium, lysed in NP-40 buffer and IRK was immunoprecipitated from the lysates with an anti-IRb antibody (Santa Cruz, Cat.# sc-711, polyclonal IgG). Prior to performing the immune complex kinase assays, the beads were equilibrated with the kinase buffer (30mM Hepes pH 7.4, 30mM NaCl, 8mM MgCl2, 4mM MnCl₂).

For HCK kinase assays, we used *HCK*-transfected COS-7 cells. The cloning and expression of HCK in COS-7 cells has been described previously (Saouaf, S. J., et al. (1995) *J. Biol. Chem.* 270, 27072-8). The pSV7c-HCK plasmid was transfected into 2 x10⁶ COS-7 cells using Lipofectamine (GIBCO/BRL), and the cells were harvested 48 hours later. The cells were lysed in NP-40 buffer and HCK was immunoprecipitated from the whole cell lysates with an anti-HCK antibody.

### Apoptosis Assays.

To induce apoptosis, cells were treated with an agonistic anti-Fas/APO-1 antibody (Bender MedSystems, City/State, Lot. 04/1295) at 0.1 µg/ml and 0.5 µg/ml final concentrations, vincristine (vincristine sulfate, USP; Pharmacia, NDC 0013-7466-86, Lot VCB019) at 10 ng/ml and 100 ng/ml final concentrations, or C2-ceramide (Biomol, Lot M8107) at 10 µM, 50 µM, and/or 100 µM final concentrations. MC540 binding (as an early marker of apoptosis) and PI permeability (as a marker of advanced stage apoptosis) were simultaneously measured in DT-40 cells 24 hours after exposure to C2-ceramide, anti-Fas, or vincristine, as previously described (Uckun, F. M., et al. (1996) *Science* 22, 1096-1100). Whole cells were analyzed using a FACStar Plus flow cytometer (Becton Dickinson, San Jose, CA). All analyses were done using 488 nm excitation from an argon laser. MC540 and PI emissions were split with a 600 nm short pass dichroic mirror and a 575 nm band pass filter was placed in front of one photomultiplier tube to measure MC540 emission and a 635 nm band pass filter was used for PI emission. In order to examine the effects of the lead BTK inhibitor on ceramide-induced apoptosis in BCR-ABL positive human ALL cell line ALL-1, cells were treated for 4 hours at 37°C with 10 µM C2-ceramide in the presence or absence of the inhibitor (200 µM LFM-A13). Subsequently, cells were washed, stained with PI and MC540, and the apoptotic fractions were determined by multiparameter flow cytometry, as described (Uckun, F. M., et al. (1996) *Science* 22, 1096-1100).

To detect apoptotic fragmentation of DNA, DT-40 cells were harvested 24 hours after exposure to anti-Fas, C2-ceramide, or vincristine. Similarly, B18.2, NALM-6, and ALL-1 cells were treated with LFM-A13 (100 µM), vincristine (VCR) (10 ng/ml), C2-Ceramide (C2-CER) (10 µM), LFM-A13 (100 µM) + VCR (10 ng/ml), LFM-A13 (100 µM) + C2-CER (10 µM) for 24 hours at 37°C. DNA was prepared from Triton-X-100 lysates for analysis of fragmentation (Uckun, F. M., et al. (1996) *Science* 22, 1096-1100). In brief, cells were lysed in hypotonic 10 mmol/L Tris-HCl (pH 7.4), 1 mmol/L EDTA, 0.2% Triton-X-100 detergent; and subsequently centrifuged at 11,000 g. To detect apoptosis-associated DNA fragmentation, supernatants were electrophoresced on a 1.2% agarose gel, and the DNA fragments were visualized by ultraviolet light after staining with ethidium bromide.

### BTK is an Anti-Apoptotic Enzyme.

The anti-apoptotic activity of BTK was evaluated by comparing the effects of the apoptosis-inducing agents C2-ceramide, vincristine, and anti-Fas monoclonal antibody on wild-type DT-40 chicken B lymphoma cells to those on a BTK-deficient subclone of DT-40 cells that was established by homologous recombination knockout (Uckun, F. M., et al. (1996) *Science* 22, 1096-1100). Ceramide, the product of ceramide synthase and sphingomyelinase, has been shown to function as a second messenger that transmits membrane-induced apoptotic signals, including the Fas-mediated and TNF receptor-mediated death signals, to downstream effectors (Enari, M., Hase, A., and Nagata, S. (1995) *EMBO J.* 14, 5201-5208). The use of vincristine, a commonly used anti-leukemia/lymphoma drug, results in a time-dependent accumulation of ceramide in treated cells that leads to apoptosis. On agarose gels, DNA from Triton-X-100 lysates of anti-Fas treated BTK-deficient DT-40 cells showed a ladder-like fragmentation pattern consistent with apoptosis, whereas no DNA fragmentation was observed in wild-type DT-40 cells (Figure 9A, Lane 7 vs Lane 14). Thus, the anti-Fas antibody treatment caused apoptosis in BTK-deficient DT-40 cells, but not in wild-type DT-40 cells consistent the fact that BTK is an inhibitor of the Fas-associated death inducing signaling complex (DISC) (Vassilev, A., et al. (1998) *J. Biol. Chem.,* 274, 1646-1656). Notably, treatment of BTK-deficient DT-40 cells with C2-ceramide (= N-acetylspingosine; a synthetic cell-permeable ceramide analog) or vincristine was able to recapitulate the effects of anti-Fas in inducing oligonucleosomal DNA fragmentation on agarose gel electrophoresis, whereas wild-type DT-40 cells were resistant to both agents, confirming the function of BTK as a negative regulator of apoptosis (Figure 9A).

In order to examine the participation of the various domains of BTK in its anti-apoptotic function, we introduced wild-type human *BTK* gene as well as human *BTK* genes harboring mutations either in the catalytic domain (Arg⁵²⁵ to Gln), SH2 domain (Arg³⁰⁷ to Ala), or PH domain (Arg²⁸ to Cys) into the BTK-deficient DT-40 cells (Enari, M., Hase, A., and Nagata, S. (1995) *EMBO J.* 14,5201-5208). As evidenced in Figure 9B, BTK-deficient DT-40 cells reconstituted with wild-type human *BTK* gene (WT) did not undergo apoptosis after treatment with C2-ceramide (Lanes 2-4) or vincristine (Lanes 8-10), whereas DT-40 subclones expressing human BTK with mutations in the kinase (K-) (Lanes 5-7 and 11-13), SH2 (mSH2) (Lanes 15-17 and 21-23), or PH (mPH) domains (Lanes 18-20 and 24-26) did. Thus, the kinase, SH2, and PH domains of BTK are important for its anti-apoptotic function.

### Homology Model of BTK Kinase Domain.

The three-dimensional coordinates of BTK used in the protein/inhibitor modeling studies were constructed based on a structural alignment with the sequences of known crystal structures for four protein kinase domains (kinase domains of HCK, FGFR, IRK, and cAPK), as detailed above (Sicheri, F., Moarefi, I., and Kuriyan, J. (1997) *Nature* 385(6617), 602-9; Mohammadi, M., et al. (1997) *Science* 276(5314), 955-60; Hubbard, S. R. (1997) *The E. M. B. O. Journal* 16(18), 5572-5581; Zheng, J., et al. (1993) *Acta Cryst.* D49, 362-365). The modeled BTK kinase domain (Figure 10A) has the expected protein kinase fold with the catalytic site in the center dividing the kinase domain into two lobes. It is composed of a smaller N-terminal lobe connected by a flexible hinge to a larger C-terminal lobe. The N-terminal lobe is rich in β-strands, while the C-terminal region is mostly helical. The catalytic site is defined by two β-sheets that form an interface at the cleft between the two lobes. It is in this catalytic region where small molecule inhibitors can bind. Our modeling studies revealed that the catalytic site of the BTK kinase domain is composed of a distinct planar rectangular binding pocket near the hinge region. The rectangular binding region is defined by residues Leu⁴⁶⁰, Tyr⁴⁷⁶, Arg⁵²⁵ and Asp⁵³⁹ that occupy the comers of the rectangle. The dimensions of this rectangle are approximately 18Å x 8Å x 9Å x 17Å and the thickness of the pocket is approximately 7Å (Figure 10B). The far left comer of the rectangle can be visualized as beginning close to the hinge region at Leu⁴⁶⁰ (shown in yellow, Figure 10B) and extending 8 Å towards the upper right to Asp⁵³⁹ (shown in blue, Figure 10B). This is the shortest side of the binding pocket and is located closer to the inner core of the protein. The left side of the pocket, which is the longest, extends from Leu⁴⁶⁰ and traces 18 Å along the hinge region up to Tyr⁴⁷⁶ (shown in green, Figure 10B). The right side of the rectangular pocket, opposite to the hinge region, extends about 9 Å from Asp⁵³⁹ to Arg⁵²⁵ (shown in pink, Figure 10B), which is immediately adjacent to the binding subsites for the sugar and triphosphate groups of ATP. The hinge region of the binding site is composed of residues 472 to 481. The solvent exposed or fourth side of the rectangle extends 17 Å along the slot-shaped opening to the catalytic site from Tyr⁴⁷⁶ to Arg⁵²⁵. The binding pocket is wider at the solvent accessible region, it narrows towards the innermost region of the binding site, and overall it is relatively shallow with a thickness of about 7 Å. The volume of the pocket is approximately 500Å³.

While most of the catalytic site residues of the BTK kinase domain were conserved relative to other tyrosine kinases, a few specific variations were observed. Residues Asn⁵²⁶ and Asp⁵³⁹ (opposite the hinge) are conserved in EGFR, IRK, HCK, and BTK. Residue Thr⁴⁷⁴ in the hinge region changes to Met in IRK, JAK1 and JAK3 and residue Tyr⁴⁷⁶ in the hinge region changes to Leu in EGFR and IRK. Residue Ser⁵³⁸ of BTK is not conserved in other kinases, but changes to Gly in JAK1 and IRK, to Thr in EGFR, and to Ala in FGF-Receptor, JAK3, and HCK. One region of the binding site contains Cys⁴⁸¹ in BTK that is more hydrophobic than the corresponding residue of PDGF-Receptor (Asp), FGF-Receptor (Asn), and IRK (Asp). These residue identity differences provide a basis for designing selective inhibitors of the BTK kinase domain.

### Structure-Based Design and Synthesis of LFM Analogs with Potent BTK-Inhibitory Activity.

In modeling studies aimed at identifying LFM analogs with a high likelihood to bind favorably to the catalytic site of the kinase domain of BTK, we chose to evaluate the estimated Kᵢ values which quantitate predicted binding interactions between the inhibitor and residues in the catalytic site of BTK. Each of the small molecule LFM analogs was individually modeled into the catalytic site of the BTK kinase domain using an advanced docking procedure (see Experimental Procedure described above). The position of quercetin in the HCK crystal structure (Sicheri et al., 1997, *Nature* 385:602) was used as a template to obtain a reasonable starting point for the docking procedure. The various docked positions of each LFM analog were qualitatively evaluated using a scoring procedure and consequently compared with the IC₅₀ values of the compounds in cell-free BTK inhibition assays. Table I shows the interaction scores, calculated Kᵢ values and measured IC₅₀ values for LFM and its analogs with BTK, the data for LFM, LFM-A1 to LFM-A12, and LFM-A14 being included for comparative purposes only.

The inhibitors in our modeling studies were sandwiched by two regions of mostly hydrophobic residues. The region above the docked inhibitor consisted of residues Leu⁴⁰⁸, Val⁴¹⁶, and Lys⁴³⁰, and the residues below the docked inhibitor included BTK residues Leu⁵²⁸, Ser⁵³⁸, Gly⁴⁸⁰, and Cys⁴⁸¹. Of all the reported compounds evaluated in our modeling studies (Table 1), we predicted that compound LFM-A13 would provide the strongest binding to BTK. The positions of the critical residues in the active site of the BTK and the docked position of the compound LFM-A13 is shown in Figure 11. Of all the possible orientations of this molecule bound to the catalytic site, the one shown in Figure 11 showed the highest interaction score with BTK. This high interaction score is indicative of an energetically favored binding mode, with a correspondingly low calculated Kᵢ value of 1.4 TM. This binding position of LFM-A13 is such that the aromatic ring of the inhibitor faces the Tyr⁴⁷⁶ residue and the flexible side chain extends towards the Asp⁵³⁹ and Arg⁵²⁵ residues. The aromatic ring is also sandwiched between the hydrophobic residues Leu⁴⁰⁸ and Val⁴¹⁶ above, and Gly⁴⁸⁰ and Leu⁵²⁸ below. Residue Ser⁵³⁸ lies below the flexible side chain of the inhibitor and the end of the side chain is located between residues Asp⁵³⁹ and Arg⁵²⁵. This position closely resembles that of the ATP analog position found in the IRK complex crystal structure (Hubbard, EMBO J, 16(18):5572-5581, 1977). According to our modeling studies, the 03 atom in the hydroxyl group of LFM-A13 would form a hydrogen bond with Asp539:O and its 04 atom would form a hydrogen bond with Arg525:N.

Figure 12 illustrates the superimposed docked positions of LFM-A13 in the catalytic site of BTK, together with compounds LFM and LFM-A12 for comparison. The molecules LFM and LFM-A12 are docked such that they lie along the hinge region, corresponding to the quercetin position in the HCK crystal structure. The aromatic ring of these molecules is close to Tyr⁴⁷⁶, and the end of the side chain is sandwiched between residues Asp⁵³⁹ and Thr⁴⁷⁴. The CF₃ group in these molecules points toward the solvent accessible region and is surrounded by Leu⁴⁰⁸ above and Gly⁴⁸⁰ below. The OH group of LFM is hydrogen-bonded to an oxygen atom of Asp⁵³⁹, and for LFM-A12, the same group is hydrogen bonded to an oxygen atom of Thr⁴⁷⁴. All LFM analogs listed in Table 1, except LFM-A13; lie along the hinge region like LFM or LFM-A12 and their side chains are sandwiched between Asp⁵²⁵ and Thr⁴⁷⁴.

A comparison of the docked positions of LFM, LFM-A12, and LFM-A13 in the BTK active site shows that although the aromatic portion of the three molecules are roughly in the same region (which is also true for the other inactive LFM analogs), the side chain of LFM-A13 is tilted away from those of the others and is sandwiched between residues Asp⁵³⁹ and Arg⁵²⁵. This rotation is likely due to a more favorable orientation of the 2,5-dibromo groups of LFM-A13. This slightly tilted position and the larger Br groups afford two advantages for the interaction of LFM-A13 with the active site residues of BTK.

The first advantage is that LFM-A13 is able to form two hydrogen bonds with active site residues Asp⁵³⁹ and Arg⁵²⁵, whereas the inactive LFM analogs form only one hydrogen bond each with the Thr⁴⁷⁴ or Asp⁵³⁹ of BTK. The second advantage for binding is the higher contact area of LFM-A13 with active site residues of BTK, relative to the other 12 inactive LFM analogs, which leads to a greater hydrophobic interaction for LFM-A13. This feature is reflected by the correspondingly higher lipophilic score for LFM-A13 in Table 1.

The results from the modeling studies discussed above prompted the hypothesis that LFM-A13 would exhibit potent BTK-inhibitory activity. In order to test this hypothesis and validate the predictive value of the described BTK homology model, we synthesized LFM-A13, LFM, and 11 other LFM analogs listed in Table 1. Again, LFM and all analogs other than LFM-A13 are synthesized and analyzed for comparative purposes only. The structures of LFM, LFM-A12, and LFM-A13 were determined by single crystal X-ray diffraction. All structures were found to have a planar conformation and all bond lengths and angles were in the expected range.

Figure 13 shows an ORTEP representation of the compound LFM-A13. The crystal structure of LFM-A13 showed that its molecular conformation was very similar to the energy-minimized molecular coordinates that were generated and used for docking studies with BTK. This conformational similarity with the crystal structures indicated that the molecular models used for docking were appropriate for modeling studies.

### Specific Inhibition of BTK by LFM-A13.

Cell-free immune complex kinase assays were used to compare the effects of LFM and 12 LFM analogs on the enzymatic activity of human BTK immunoprecipitated from B18-2 cells (Uckun, F. M., et al. (1996) *Science* 22, 1096-1100) (i.e., BTK-deficient DT-40 chicken lymphoma B-cells reconstituted with wild-type human BTK gene). As shown in Table 1, only LFM-A13 exhibited significant BTK inhibitory activity with an IC₅₀ value of 6.2 ± 0.3 µg/ml (= 17.2 ± 0.8 µM). None of the other compounds, which were included for comparison only, inhibited BTK even at concentrations as high as 100 µg/ml (i.e., at a range of 349 µM for LFM-A12 to 495 µM for LFM-A14).

LFM-A13 was also effective against recombinant BTK expressed in a baculovirus vector expression system with an IC₅₀ value of 0.9 Tg/ml (~ 2.5 TM, Figure 14A), as well as BTK immunoprecipitated from NALM-6 human B-lineage ALL cell lysates (Figure 14B). Furthermore, treatment of B18.2 cells (Figure 14C) or NALM-6 cells (data not shown) with LFM-A13 resulted in a dose-dependent inhibition of cellular BTK activity. The inhibitory activity of LFM-A13 against BTK was specific since it did not affect the enzymatic activity of other protein tyrosine kinases, including Janus kinases JAK1 and JAK2, Src family kinase HCK, and receptor family tyrosine kinase IRK, at concentrations as high as 100 µg/ml (~278 µM; Table 2, Figure 15).

### Structural Basis for the BTK-Specificity of LFM-A13.

Biological assays have shown LFM-A13 to be a selective inhibitor of BTK, whereas it is a poor inhibitor of EGFR, HCK, JAK1, JAK3 and IRK. To evaluate this selectivity we constructed a homology model of EGFR, JAK1 and JAK3 using homologous crystal structure coordinates of protein kinases IRK, HCK, and cAPK as a template. The models were then used to study the binding of small molecules such as LFM-A13 into the catalytic sites of these kinases, and to better understand how LFM-A13 can inhibit BTK but not EGFR, IRK, JAK1, JAK3 or HCK. These studies identified three factors that may contribute to the specificity of LFM-A13 for BTK.

The small molecule LFM-A 13 was docked into the kinase domains of IRK, HCK, JAK3 and EGFR. Table 2 shows the interaction scores, calculated Kᵢ values, and measured IC₅₀ data for LFM-A 13 with these kinases. We postulate that the selectivity of LFM-A13 for BTK results from favorable interactions with the BTK catalytic site residues that are not present in the other kinases studied. There are some residues in the BTK active site that differ from those of other PTKs. These differences are illustrated in Figure 16 that shows the backbone of the BTK catalytic site, the residue differences between BTK and other kinases, and the docked positions of LFM-A13 in the kinase domains of BTK, HCK, JAK3, JAK1, EGFR, and IRK.

It is believed that the residue differences shown at positions A, B, and C in Figure 16 may contribute to the specificity of LFM-A13 for BTK. Kinases that are not inhibited by LFM-A13, such as IRK (gray) and JAK1/JAK3 (pink) contain a methionine residue at position A which protrudes into the active site and prevents the close contact of small molecules like LFM-A13 with the hinge region of the binding site. As a result, LFM-A13 can lose favorable hydrophobic contact with the hinge region of the kinase domains of these proteins and does not bind to it tightly. Moreover, docking studies indicated that the favorable position of LFM-A13 in the BTK kinase domain is such that the side chain of the small molecule is located between residues Asp⁵³⁹ and Arg⁵²⁵, and forms hydrogen bonds with them.

In addition, an aromatic residue at position B of BTK increases the hydrophobic interaction of the LFM-A13 molecule with the receptor, an interaction which is lost in EGFR (red, Figure 16) and IRK (dark blue, Figure 16). While the Lipo scores ranged between 457 and 473 for other kinases, the Lipo score for BTK was higher (more favorable) at 517. Finally, the Arg⁵²⁵ residue at position C of BTK can hydrogen bond to LFM-A13. This interaction is lost in HCK (yellow), which contains an Ala at the C position. The favorable position of LFM-A13 at the HCK kinase domain (shown in yellow) is such that the small molecule is aligned along the hinge region. At this position LFM-A13 does not form hydrogen bonds with HCK, which is not the case for BTK. The longer side chain of Arg⁵²⁵ in BTK (position C) is involved in hydrogen bonding with LFM-A13, whereas HCK has an Ala at this position that is not able to form the same hydrogen bond.

### LFM-A13 Enhances the Sensitivity of B-lineage Acute Lymphoblastic Leukemia (ALL) Cells to Ceramide- and Vincristine-Induced Apoptosis.

Patients with Philadelphia Chromosome (Ph+) ALL have a dismal outcome after intensive multimodality treatment programs. The treatment failure of these patients could be overcome if the apoptotic threshold of their leukemic cells could be decreased. We set out to determine if LFM-A13, by means of inhibiting the anti-apoptotic tyrosine kinase BTK, could alter the sensitivity of the Ph+ ALL cell line ALL-1 to C2-ceramide. As shown in Figure 17, treatment with LFM-A13 significantly enhanced the chemosensitivity of ALL-1 cells to ceramide-induced apoptosis, as evidenced by a greater percentage of cells treated with LFM-A13 plus C2-ceramide, as compared to cells treated with C2-ceramide alone or LFM-A13 alone, showing dual PI/MC540 fluorescence (shown in blue color) consistent with advanced stage apoptosis. Furthermore, on agarose gels, DNA from Triton-X-100 lysates of B18.2 chicken lymphoma B cells (i.e., BTK-deficient DT40 cells reconstituted with wild-type human BTK gene; see also Figure 9), NALM-6 human pre-B ALL cells, and ALL-1 cells showed a ladder-like fragmentation pattern consistent with apoptosis after treatment with LFM-A13 plus ceramide or LFM-A13 plus vincristine, which was more pronounced than after treatment with LFM-A13, ceramide, or vincristine alone (Figure 18). These results demonstrated that LFM-A13 enhances the sensitivity of B-lineage leukemia/lymphoma cells to both ceramide-induced and vincristine-induced apoptosis.

### Example 4. Modifications on DDE LFM-A13 for BTK Inhibition

The following example describes how the above homology model and information relating to differences between the binding domain of BTK and other protein tyrosine kinases was used to identify additional compounds of formula I that are specific BTK inhibitors.

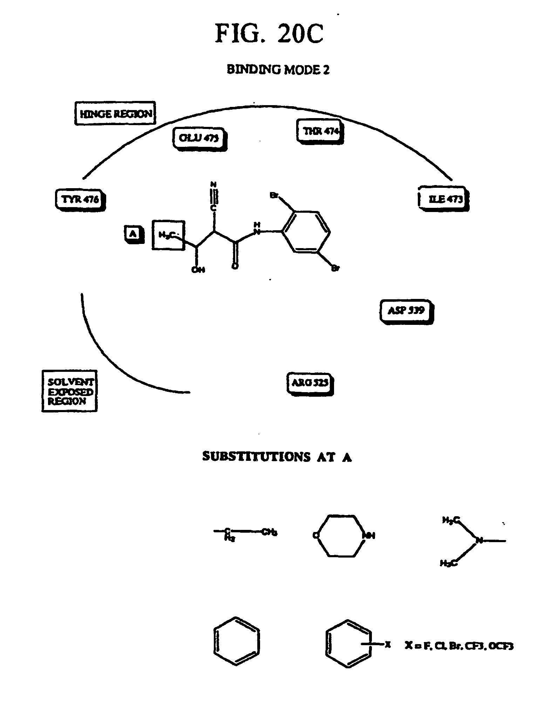

Structural and chemical features of LFM analogs that are proposed to aid binding to the BTK catalytic site are described below and illustrated in Figures 20A-20C. Binding Mode 1, (Figures 20A-20B) shows the most likely mode of binding of the lead compound LFM-A13 at the BTK catalytic site. Based on the modifications of the lead compound, a second mode of binding may also be possible, illustrated in Figure 20C (Binding Mode 2).

Table 3 shows the residue differences that the ATP binding site between the ten PTK's: EGFR, Btk, Hck, Jak1, Jak3, IR, FGFR1, PDGFRϑ, VEGFR2 and Src.

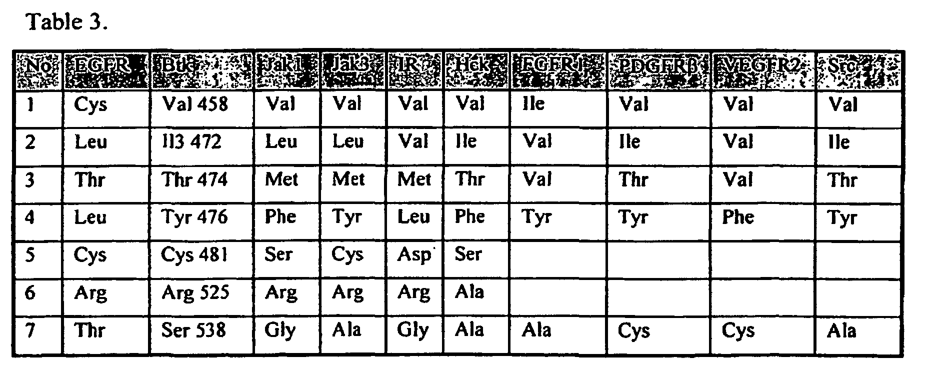

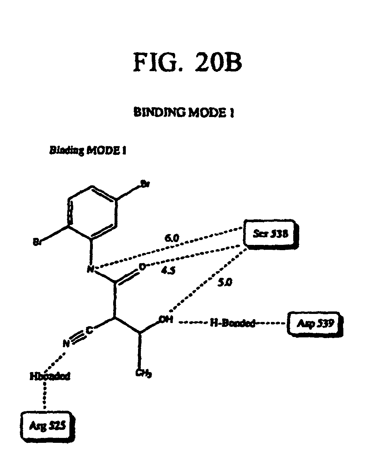

Comparison of the inhibitor binding pocket of BTK with that of EGFR, Hck, Jak1, Jak3, IR, FGFR1, PDGFRβ, VEGFR2, and Src shows that SER 538 in the ATP binding cleft of BTK is unique (Table 3), and a target for specific BTK inhibitors design. Ligands designed to interact with this residue should have increased specificity for BTK. Figure 20B shows the distances (in angstroms) of the NH, =O, and OH groups of the docked LFM-13 ligand from SER 538. When looking into the BTK binding cleft with the aromatic ring of the ligand facing out and the chain going inward, this residue is located below the ligand. Non-planar, single chain substitutions ending with O or N and having the appropriate lengths (Figure 20B) at the NH, =O, and OH groups should allow the formation of hydrogen bonds with Ser 538. Based on this observation and on modeling and biological data for the 13 leflunomide metabolite analogs described above, additional compounds of formula I can be designed to better interact with the binding pocket that are expected to be inhibitors of BTK.

### Example 5. Second Generation LFM analogs

The following example describes how novel compounds of formula I designed to target SER 538 are designed and prepared.

Using the design parameters described in Figures 20A-20B, a second generation of LFM analogs (LFM- A15-20) were designed and synthesized. The compounds have the following structural formula: 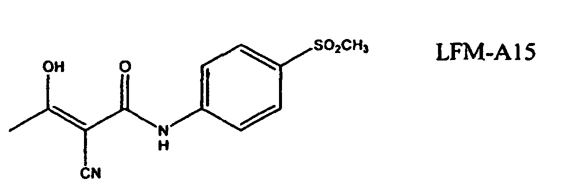 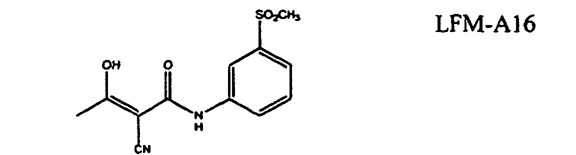 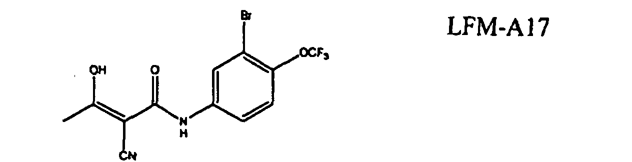 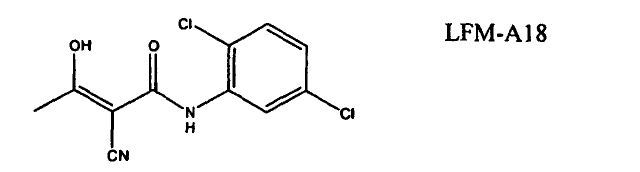 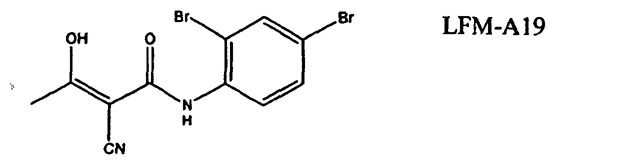 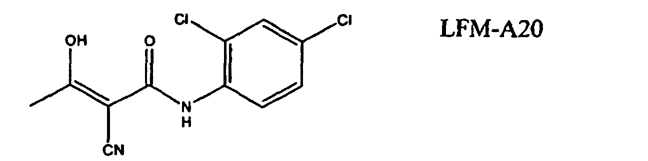 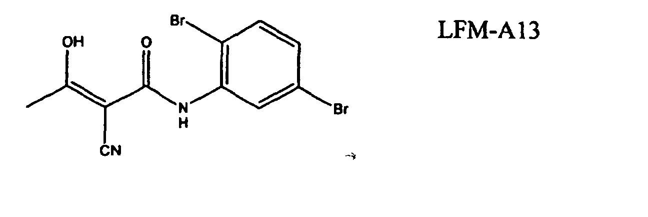 The following synthetic scheme and that shown in figure 19 were used to generate the compounds: 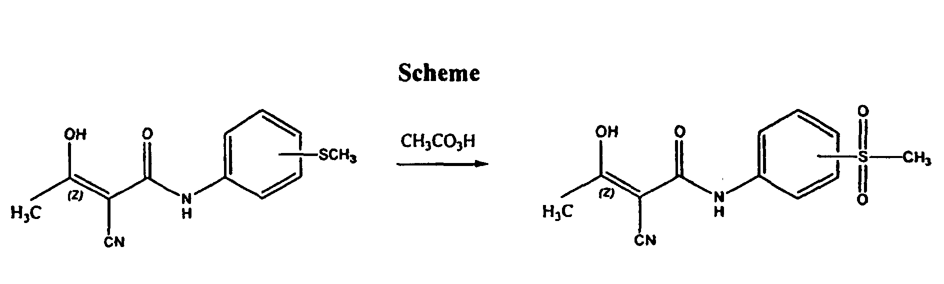 **Synthetic Procedure A.** 1,3-Diisopropylcarbodiimide (1.75 g; 13.9 mmol) was added to a solution of cyanoacetic acid 1 (1.70 g; 20.0 mmol) and the desired substituted -aniline 2 (12.6 mmol) in tetrahydrofuran (25 mL) at 0°C. The mixture was stirred for 12 hours at room temperature. The urea precipitate (reaction side product) was removed by filtration and partitioned between ethyl acetate and 0.5 N HCl. The organic layer was sequentially washed with brine twice, dried over anhydrous Na₂SO₄ and concentrated by rotary-evaporation. Finally, the crude solid product was recrystallized from ethyl alcohol to give pure 3. Sodium hydride (0.93 g; 60% in mineral oil; 23.2 mmol) was added slowly to the solution of 3 (12.0 mmol) in tetrahydrofuran (40 mL) at 0°C. After stirring for 30 minutes at 0°C, acetyl chloride (1.04g; 13.2 mmol) was added to the reaction mixture. The reaction was continued for another hour and then was quenched by the addition of acetic acid (2 mL). The mixture was poured into ice water (100 mL) containing 2.5 mL of hydrochloric acid to precipitate the crude product, which was collected by filtration and washed with water. The pure product was obtained by recrystallization.
**Synthetic Procedure B.** α-Cyano-β-hydroxy-β-methyl-*N* -[(methylthio)phenyl]propenamide (2.48g, 10.0 mmol) was dissolved in acetic acid (150 mL), and peracetic acid (8.6 mL of 32% wt solution in acetic acid) was added. The mixture was stirred overnight at room temperature, and water (75 mL) was added. The precipitate was filtered and washed with water. The pure product was obtained by recrystallization.

### Physical data for synthesized compounds:

α-Cyano-β-hydroxy-β-methyl-*N*-[4-(methylsulfonyl)phenyl]propenamide. Melting point: 205-206 °C. ¹H NMR (DMSO-*d*₆): δ 11.26 (s, 1H, NH), 7.81 (d, *J* = 9.0 Hz, 2H, ArH), 7.76 (d, 9.0 Hz, 2H, ArH). 3.15 (s, 3H, SO₂CH₃). IR (KBr): 3309, 2225. 1643 and 1586 cm⁻¹. MS (EI): *m*/*z* 281.0 (M+H⁺), 172.1.
α-Cyano-β-hydroxy-β-methyl-*N*-[3-(methylsulfonyl)phenyl]propenamide. Melting point: 213-214 °C. ¹H NMR (DMSO-*d*₆): δ 10.98 (s, 1H, NH), 8.18 (m, 1H, ArH), 7.82 (m, 1H, ArH), 7.60 (m, 2H, ArH), 3.18 (s, 3H, SO₂CH₃). IR (KBr): 3278, 2231, 1607 and 1555 cm⁻¹. MS (EI): *m*/*z* 281.0 (M + H⁺), 172.1.
α-Cyano-β-hydroxy-β-methyl-*N*-[3-bromo-4-(trifluoromethoxy)phenyl]-propenamide. Melting point: 178-179 °C. ¹H NMR (DMSO-*d*₆): δ 11.03 (s,1H, NH), 8.12 (d, *J* + 2.4 Hz, 1H, ArH), 7.55 (dd, *J* = 2.4, 9.0 Hz, 1H, ArH). 7.45 (m, 1H, ArH), 5.53 (s br, 1H, OH). 2.20 (s, 3H, CH₃). IR (KBr): 3304, 2350, 1620 and 1602 cm⁻¹. MS (EI): *m*/*z* 365.0 (M+H)⁺, 225.9.
α-Cyano-β-hydroxy-β-methyl-*N*-(2,4-dibromophenyl)propenamide. Melting point: 181-182 °C. ¹H NMR (DMSO-*d*₆): δ 11.03 (s, 1H, NH), 8.14 (m, 1H, ArH), 7.84 (d, *J =* 2.4 Hz, 1H, ArH), 7.51 (dd, *J =* 2.4, 9.0 Hz, 1H, ArH), 5.65 (s br, 1H, OH), 2.20 (s, 3H, CH₃). IR (KBr): 3360, 2205, 1591 and 1530 cm⁻¹. MS (EI): *m*/*z* 361.0 (M + H⁺), 249.9.
α-Cyano-β-hydroxy-β-methyl-*N*-(2,4-dichlorophenyl)propenamide. Melting point: 143-144 °C. ¹H NMR (DMSO-*d*₆): δ 11.23 (s, 1H, NH), 8.29 (m, 1H, ArH), 7.6 (d, *J =* 2.4 Hz, 1H, ArH). 7.35 (dd, *J =* 2.4, 9.0 Hz, 1H, ArH), 5.17 (s br, 1H, OH), 2.18 (s, 3H, CH₃). IR (KBr): 3371, 2210, 1601 and 1540 cm⁻¹. MS (EI): *m*/*z* 271.0 (M⁺), 162.0.
α-Cyano-β-hydroxy-β-methyl-*N*-(2,5-dichlorophenyl)propenamide. Melting point: 143-144 °C. ¹H NMR (DMSO-*d*₆): δ 11.70 (s, 1H, NH), 8.51 (d, *J =* 3.0 Hz, 1H, ArH), 7.45 (d, *J* = 8.7 Hz, 1H, ArH), 7.05 (dd, *J* = 3.0, 8.7 Hz, 1H, ArH), 4.97 (s br, 1H, OH), 2.14 (s, 3H, CH₃). IR (KBr): 3402, 2205, 1627 and 1606 cm⁻¹. MS (EI): *m*/*z* 271 (M⁺), 162.0.

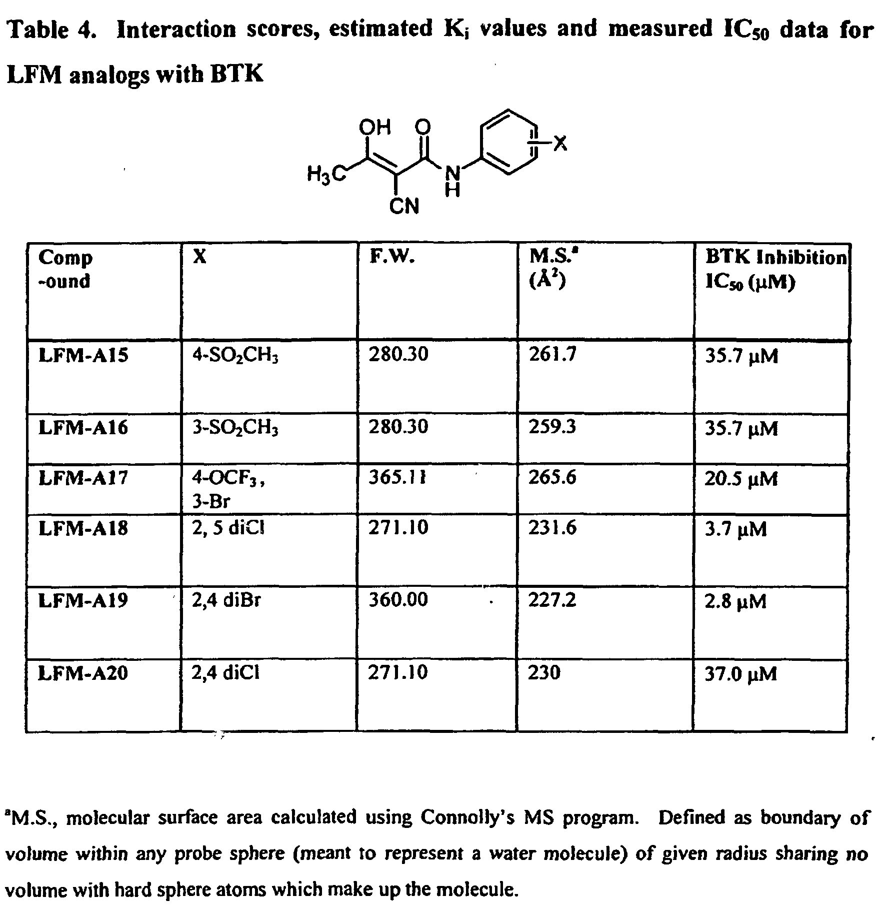

The second generation LFM analogs were evaluated for interaction with the BTK binding pocket model, and evaluated for inhibitory activity against BTK as described in the Example above. The data are shown in Table 4, and demonstrate these novel compounds to be potent inhibitors of BTK.

### Example 7: Novel BTK Inhibitor Designs

Figure 21 shows the binding features common to 5,7-dihydroxy-8-propanoyl-4-propyl-2H-1-benzopyran-2-one (for comparison only) and LFM-A13, based on docking the compounds into the ATP-binding site of BTK. Both inhibitors contain hydrogen-bonding groups (OH, CN) positioned to interact with Arg 525 and Asp 539. Compound designs that better fill the binding pocket and interact favorably with eligible residues in the pocket are designed.

These compounds, due to their increased volume and groups designed to interact with the binding pocket residue, are expected to have potent BTK-inhibitory activity.

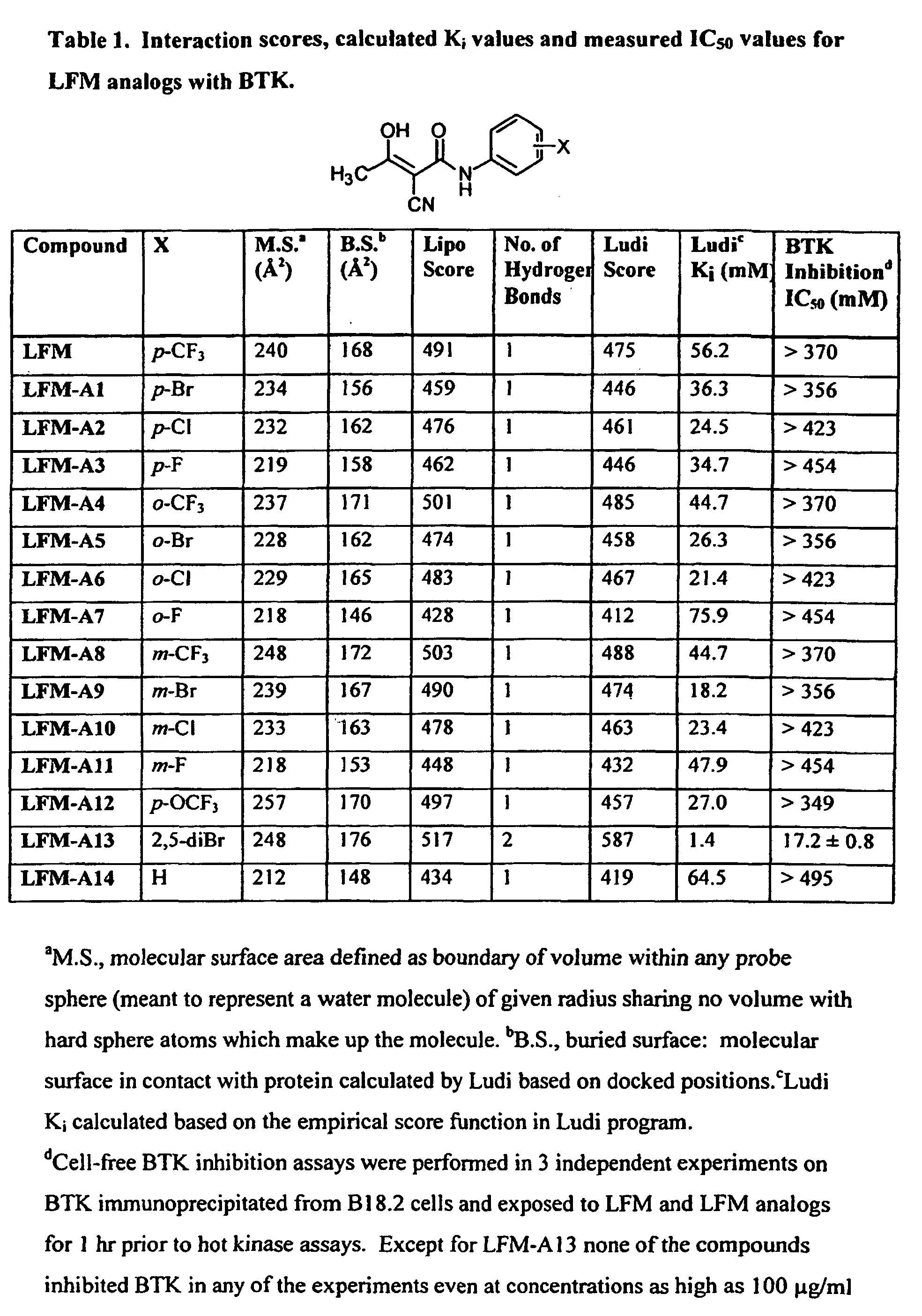

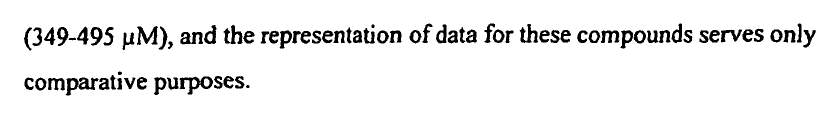

**Table 2.**

| **Interaction scores, calculated K**_{**i**} **values and measured IC**_{**50**} **values for LFM-13 with several different PTKs.** | | | | | | | |
|---|---|---|---|---|---|---|---|
| **Tyrosine Kinase** | **M.S.**^{**a**} **(Å**^{**2**}**)** | **B.S.**^{**b**} **(%)** | **Lipo Score** | **No. of Hydrogen Bonds** | **Ludi Score** | **Ludi**^{**c**} **K**_{**i**} **(TM)** | **Inhibition IC**_{**50**} **(TM)** |
| **JAK1** | 250 | 68 | 497 | 0 | 396 | 110 | > 278 |
| **JAK3** | 246 | 67 | 484 | 0 | 383 | 148 | > 278 |
| **IRK** | 248 | 64 | 466 | 1 | 450 | 31.6 | > 278 |
| **EGFR** | 248 | 66 | 479 | 0 | 378 | 166 | > 278 |
| **HCK** | 246 | 65 | 468 | 0 | 367 | 214 | > 278 |

| | | | | | | | |
|---|---|---|---|---|---|---|---|
| ^{a}M.S., molecular surface area calculated as boundary of volume within any probe sphere (meant to represent a water molecule) of given radius sharing no volume with hard sphere atoms which make up the molecule. | | | | | | | |
| ^{b}B.S., buried surface: percentage of molecular surface in contact with protein calculated by Ludi based on docked positions. | | | | | | | |
| ^{c}Ludi Kᵢ calculated based on the empirical score function in Ludi program. ^{d}Cell-free tyrosine kinase inhibition assays were performed in 2-3 independent experiments, as described in the Methods section. LFM-A13 did not inhibit JAK1, JAK3, IRK, EGFR, or HCK in any of the experiments even at concentrations as high as 100 µg/ml (278 µM). The results from a representative experiment are depicted in Figure 15. | | | | | | | |

## Claims

1. The use of a compound of formula I: 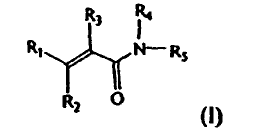 where:
R₁ is (C₁-C₃)alkyl, (C₃-C₆)cycloalkyl, phenyl, or NRₐR_{b};
R₂ is hydroxy, (C₁-C₆)alkoxy, (C₁-C₆)alkanoyloxy, amino (C₂-C₅)alkoxy; hydroxy (C₂-C₅)alkoxy, amino (C₂-C₅)alkanoyloxy; or hydroxy (C₂-C₅) alkanoyloxy;
R₃ is cyano or (C₁-C₃)alkanoyl;
R₄ is hydrogen, (C₁-C₃)alkyl; hydroxy (C₂-C₅)alkyl; or amino (C₂-C₅)alkyl;
R₅ is phenyl substituted by -S(O)₂R_{c}, or by halo and at least one other substituent;
Rₐ and R_{b} are each independently hydrogen, or (C₁-C₃)alkyl; or Rₐ and R_{b} together with the nitrogen to which they are attached are pyrrolidino, piperidino, morpholino, or thiomorpholino;
wherein any phenyl of R₁ and R₅ is optionally substituted with one or more substituents independently selected from halo, nitro, cyano, hydroxy, trifluoromethyl, trifluoromethoxy, (C₁-C₃)alkoxy, (C₁-C₃)alkyl, (C₁-C₃)alkanoyl, -S(O)₂R_{c}, and NRₐR_{b}; wherein R_{c} is (C₁-C₃)alkyl, or aryl
or a pharmaceutically acceptable salt thereof;
for the preparation of a medicament for treating a condition or a disease in which Bruton's Tyrosine Kinase activity is implicated, said condition or disease being selected from B-cell lymphoproliferative disorders/autoimmune diseases, mast cell disorders, conditions that relate to improper platelet aggregation, and rejection of xenotransplants.

2. The use according to claim 1, wherein the condition or disease in which Bruton's Tyrosine Kinase activity is implicated is allergy.

3. The use according to claim 1, wherein the condition or disease in which Bruton's Tyrosine Kinase activity is implicated is anaphylactic shock.

4. The use according to claim 1, wherein the condition or disease in which Bruton's Tyrosine Kinase activity is implicated is improper platelet aggregation.

5. The use according to claim 1, wherein R₁ is C₁₋₃ alkyl, R₂ is hydroxy and R₃ is cyano.

6. The use according to claim 1, wherein R, is methyl.

7. The use according to claim 1, wherein the compound comprises at least one of: 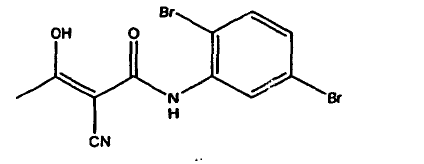 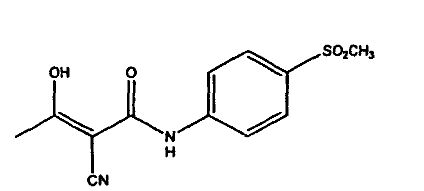 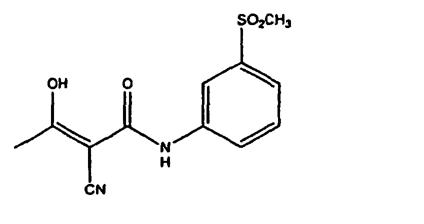 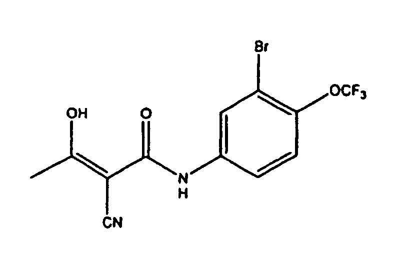 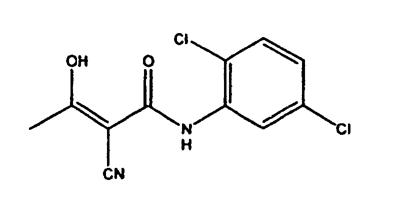 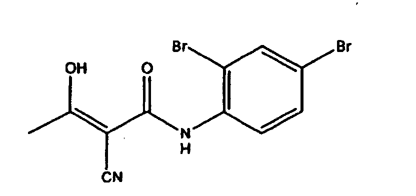 or 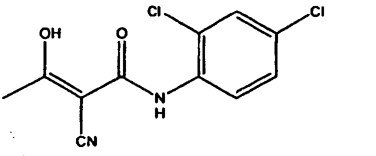 and a pharmaceutically acceptable salt thereof.

8. The use according to claim 1, wherein the compound comprises 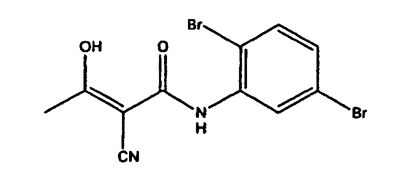 or a pharmaceutical acceptable salt thereof.

## Patentansprüche

1. Verwendung einer Zusammensetzung der Formel I: 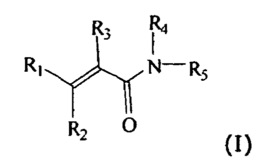 in der:
R₁ für (C₁-C₃)-Alkyl, (C₃-C₆)-Cycloalkyl, Phenyl oder NRₐR_{b} steht;
R₂ für Hydroxy, (C₁-C₆)-Alkoxy, (C₁-C₆)-Alkanoyloxy, Amino-(C₂-C₅)-alkoxy, Hydroxy-(C₂-C₅)-alkoxy, Amino-(C₂-C₅)-alkanoyloxy oder Hydroxy-(C₂-C₅)-alkanoyloxy steht;
R₃ für Cyano oder (C₁-C₃)-Alkanoyl steht;
R₄ für Hydrogen, (C₁-C₃)-Alkyl, Hydroxy-(C₂-C₅)-alkyl oder Amino-(C₂-C₅)-alkyl steht;
R₅ für durch -S(O)₂R_{c} oder Halo und mindestens einen anderen Substituenten substituiertes Phenyl steht;
Rₐ und R_{b} jeweils unabhängig voneinander für Hydrogen oder (C₁-C₃)-Alkyl stehen; oder Rₐ und R_{b} zusammen mit dem Stickstoff, mit welchem sie verbunden sind, für Pyrrolidino, Piperidino, Morpholino oder Thiomorpholino stehen;
wobei etwaiges Phenyl als R₁ und R₅ wahlweise substituiert ist durch einen oder mehreren Substituenten, die unabhängig voneinander ausgewählt sind unter Halo, Nitro, Cyano, Hydroxy, Trifluoromethyl, Trifluoromethoxy, (C₁-C₃)-Alkoxy, (C₁-C₃)-Alkyl, (C₁-C₃)-Alkanoyl, -S(O)₂R_{c} und NRₐR_{b}; wobei R_{c} für (C₁-C₃)-Alkyl oder Aryl steht;
oder ein pharmazeutisch akzeptables Salz davon;
für die Herstellung eines Medikaments zur Behandlung eines Zustandes oder einer Erkrankung, worin Brutons Tyrosin-Kinase-Aktivität eine Rolle spielt, wobei der Zustand oder die Erkrankung ausgewählt ist unter Störungen der B-Zell-Lymphozytenproliferation/Autoimmunerkrankungen, Mastzellenstörungen, Zuständen verbunden mit einer unzweckmässigen Blutplättchenaggregation und Abstossung von Xeno-Transplantaten.

2. Verwendung nach Anspruch 1, wobei der Zustand oder die Erkrankung, worin Brutons Tyrosin-Kinase-Aktivität eine Rolle spielt, eine Allergie ist.

3. Verwendung nach Anspruch 1, wobei der Zustand oder die Erkrankung, worin Brutons Tyrosin-Kinase-Aktivität eine Rolle spielt, anaphylaktischer Schock ist.

4. Verwendung nach Anspruch 1, wobei der Zustand oder die Erkrankung, worin Brutons Tyrosin-Kinase-Aktivität eine Rolle spielt, eine unzweckmässige Blutplättchenaggregation ist.

5. Verwendung nach Anspruch 1, wobei R₁ für C₁₋₃-Alkyl, R₂ für Hydroxy und R₃ für Cyano steht.

6. Verwendung nach Anspruch 1, wobei R₁ für Methyl steht.

7. Verwendung nach Anspruch 1, wobei die Zusammensetzung mindestens eine der folgenden : 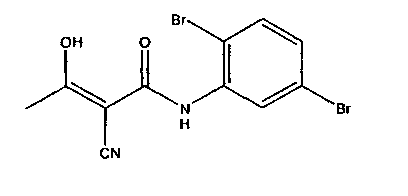 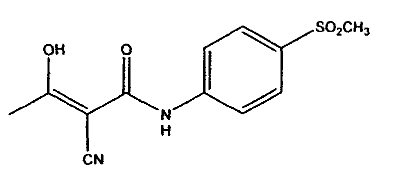 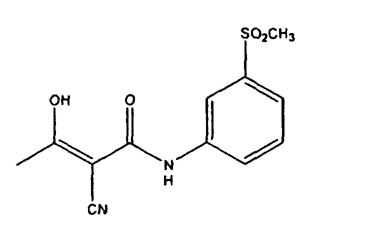 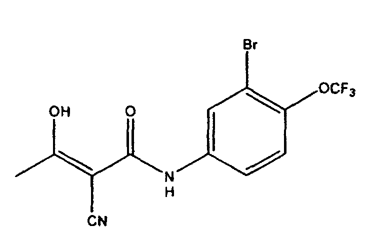 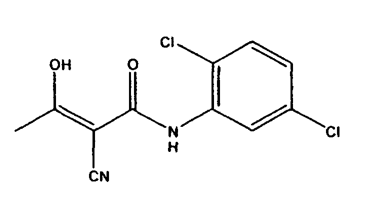 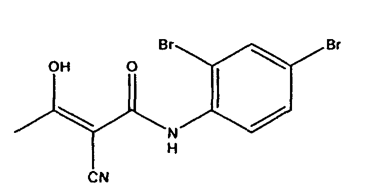 und 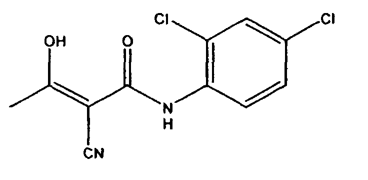 sowie ein pharmazeutisch akzeptables Salz davon umfasst.

8. Verwendung nach Anspruch 1, wobei die Zusammensetzung 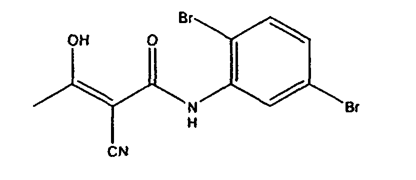 oder ein pharmazeutisch akzeptables Salz davon umfasst.

## Revendications

1. Utilisation d'un composé de formule (I) : 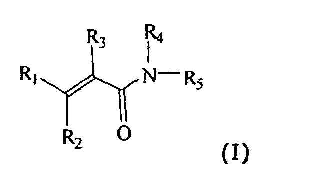 dans lequel
R₁ représente (C₁-C₃)alkyle, (C₃-C₆)cycloalkyle, phényle ou NRₐR_{b} ;
R₂ représente hydroxy, (C₁-C₆)alkoxy, (C₁-C₆)alkanoyloxy, amino-(C₂-C₅)alkoxy, hydroxy-(C₂-C₅)alkoxy, amino-(C₂-C₅)alkanoyloxy ou hydroxy-(C₂-C₅)alkanoyloxy ;
R₃ représente cyano ou (C₁-C₃)alkanoyl ;
R₄ représente hydrogène, (C₁-C₃)alkyle, hydroxy-(C₂-C₅)alkyle ou amino-(C₂-C₅)alkyle ;
R₅ représente phényle substitué par -S(O)₂R_{c} ou par halogène ainsi qu'au moins un autre substituant ;
Rₐ et R_{b} représentent indépendamment l'un de l'autre l'hydrogène ou (C₁-C₃)alkyle ; ou Rₐ et R_{b} en combinaison avec le nitrogène, auquel ils sont alliés, représentent pyrrolidino, pipéridino, morpholino ou thiomorpholino;
le phényle éventuellement présent en R₁ et en R₅ étant facultativement substitué par un ou plusieurs substituants, choisis indépendamment parmi halogène, nitro, cyano, hydroxy, trifluoromethyle, trifluoromethoxy, (C₁-C₃)alkoxy, (C₁-C₃)-alkyle, (C₁-C₃)-alkanoyl, -S(O)₂R_{c} et NRₐR_{b}, R_{c} représentant (C₁-C₃)-alkyle ou aryl;
ou l'un de ses sels pharmacologiquement acceptables;
pour la préparation d'un médicament pour le traitement d'un état ou d'une maladie impliquant l'activité tyrosine-kinase de Bruton, l'état ou la maladie étant choisi(e) parmi les syndromes lymphoprolifératifs de lymphozyte B/les maladies auto-immunes, les mastocytoses, les troubles associés à une agrégation impropre de plaquettes et le rejet de xenotransplants.

2. Utilisation selon la revendication 1, charactérisée en ce que l'état ou la maladie impliquant l'activité tyrosine-kinase de Bruton est une allergie.

3. Utilisation selon la revendication 1, charactérisée en ce que l'état ou la maladie impliquant l'activité tyrosine-kinase de Bruton est un choc anaphylactique.

4. Utilisation selon la revendication 1, charactérisée en ce que l'état ou la maladie impliquant l'activité tyrosine-kinase de Bruton est une agrégation impropre de plaquettes.

5. Utilisation selon la revendication 1, charactérisée en ce que R₁ représente C₁₋₃-alkyle, R₂ représente hydroxy et R₃ représente cyano.

6. Utilisation selon la revendication 1, charactérisée en ce que R₁ représente méthyle.

7. Utilisation selon la revendication 1, charactérisé en ce que le composé comporte au moins un des suivants : 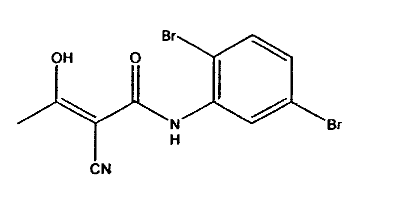 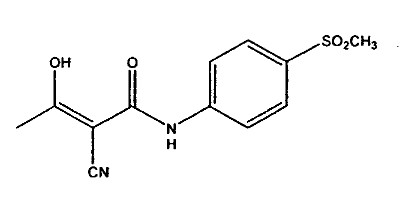 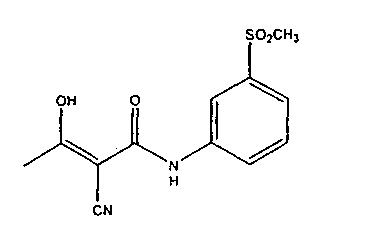 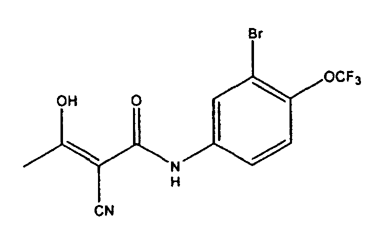 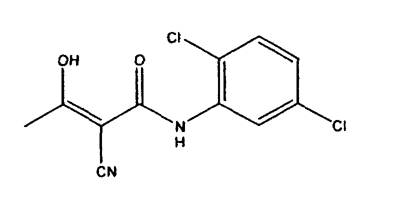 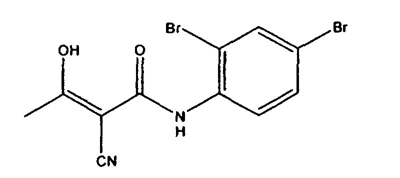 et 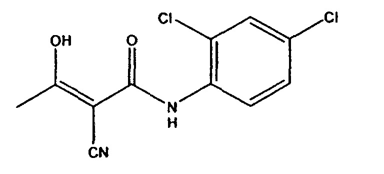 ainsi qu'un des sels pharmacologiquement acceptables de ceux-ci.

8. Utilisation selon la revendication 1, charactérisée en ce que le composé comporte 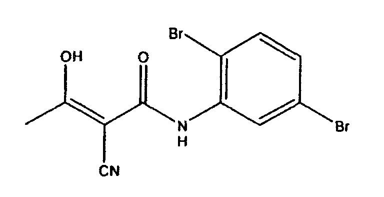 ou l'un de ses sels pharmacologiquement acceptables.
